# EUROPÄISCHE PATENTANMELDUNG

(11) **EP 3 797 675 A1**
(43) Veröffentlichungstag der Anmeldung: **31.03.2021**
(21) Anmeldenummer: 20197688.3
(22) Anmeldetag: 23.09.2020
(51) Int. Cl.: A61B 1/07, A61B 1/00, G02B 23/26, G02B 23/24, G02B 6/04, G02B 6/02, G02B 1/04

(54) **LICHTLEITER FÜR DIAGNOSE-, OPERATIONS- UND/ODER THERAPIEGERÄT**

(30) Priorität: 26.09.2019 DE 102019125912
(71) Anmelder: SCHOTT AG, 55122 Mainz (DE)
(72) Erfinder: SCHULTHEIS, Bernd, 55270 Schwabenheim (DE); CRAMER, Martin, 65187 Wiesbaden (DE); RUSSERT, Hubertus, 55270 Jugenheim (DE); WERNER, Holger, 60596 Frankfurt am Main (DE); WALTER, Lukas, 65375 Oestrich-Winkel (DE); VOIGT, Hildegard, 55283 Nierstein (DE); KAPPEL, Markus, 55595 Roxheim (DE); STEIGER, Erwin, 26683 Saterland-Ramsloh (DE)
(74) Vertreter: Blumbach · Zinngrebe Patentanwälte PartG mbB

(57) **Zusammenfassung**

Die Erfindung betrifft einen Lichtleiter zur Transmission elektromagnetischer Strahlung für ein Diagnose, Operations- und/ oder Therapiegerät zur Einbringung in den menschlichen oder tierischen Körper oder zur in-vitro Untersuchung von menschlichen oder tierischen Blutproben oder sonstigen Körperzellen, insbesondere ein Endoskop oder ein Einweg-Endoskop. Weitere Aspekte betreffen die Verwendung eines solchen Lichtleiters und ein Diagnose-, Operations- und/oder Therapiegerät mit einem solchen Lichtleiter.

## Beschreibung

Die Erfindung betrifft einen Lichtleiter zur Transmission elektromagnetischer Strahlung für ein Diagnose-, Operations- und/ oder Therapiegerät zur Einbringung in den menschlichen oder tierischen Körper oder zur in-vitro Untersuchung von menschlichen oder tierischen Blutproben oder sonstigen Körperzellen, insbesondere ein Endoskop oder ein Einweg-Endoskop, wobei der Lichtleiter jeweils eine proximale Endfläche zur Ein- bzw. Auskopplung von elektromagnetischer Strahlung und eine distale Endfläche zur Aus- bzw. Einkopplung elektromagnetischer Strahlung aufweisen.

Diagnose-, Operations- und/oder Therapiegeräte, wie beispielsweise Endoskope zur Diagnose, für minimalinvasive Eingriffe oder zur Therapie, sind als starre oder flexible Ausführungen bekannt und in der Literatur hinreichend beschrieben. Einweg-Endoskope, oder auch "disposable Endoscopes" genannt, werden heute zunehmend eingesetzt, um insbesondere die Patientensicherheit bei medizintechnische Untersuchungen, Therapien und/ oder minimal invasive Eingriffen zu erhöhen, in dem durch eine Einmal-Verwendung Kontaminationen verhindert werden. Zwar sind bisherige Endoskope dafür konzipiert, dass diese im Sinne der Medizintechnik aufbereitbar, das heißt reinigbar, sterilisierbar und vor allem autoklavierbar, sind.

Trotzdem kann es hier aufgrund falscher Anwendung der Aufbereitung bzw. ungünstigem Design derartiger Geräte vereinzelt vorkommen, dass nicht die erforderliche Keimzahlreduzierung erreicht wird, und damit Keime bei der nächsten Anwendung auf den Patienten übertragen werden können. Dies kann durch den Einsatz von derartigen Einweg-Endoskopen verhindert werden.

Ein weiterer Aspekt für den vermehrten Einsatz von Einweg-Endoskopen stellt auch eine Wirtschaftlichkeitsbetrachtung dar. Insbesondere der ordnungsgemäß und regelmäßig nach jeder Behandlung durchgeführte Aufbereitungsprozess erfordert inzwischen hohe Kosten beim praktizierenden Arzt oder in der Klinik. Zudem sind hohe Investitionen für Reinigungsgeräte, wie Thermodesinfektoren, und Autoklaviergeräten und/ oder Plasma-Sterilisationsgeräte erforderlich, so dass insgesamt der Einsatz derartiger Einweg-Endoskope gerechtfertigt ist.

Ein weiterer Vorteil ergibt sich daraus, dass derartige Einweg-Endoskope zum einen mobil als "hand-held"-Geräte einsetzbar sind und daher auch in der Notfall-Medizin, im Militär-Sanitätseinsatz oder auch in schwer zugänglichen Regionen, zum Beispiel auch bei Katastropheneinsätzen, einsetzbar sind, wo insbesondere keine Aufbereitungsmöglichkeiten zur Verfügung stehen.

Derartige Einweg-Endoskope, "Single-Use"-Endoskope oder "disposable Endoscopes", wie sie in der Literatur beschrieben sind, sind beispielhaft in folgenden Schriften beschrieben:
Die Schrift US 3581738 A1 offenbart ein Einweg-Endoskop, umfassend einen Körper aus synthetischem Harzmaterial mit einer im allgemeinen rohrförmigen Seitenwand, die ein Spekulum bildet, und ein einheitliches längliches Lichtleitelement, das in die Seitenwand eingebettet ist, wobei das Element aus einem Lichtleitmaterial gebildet ist, das mit einem transparenten Material mit einem Brechungsindex, der sich von dem des Lichtleitmaterials unterscheidet, überzogen ist, wobei der Körper aus zwei sich axial vom Endoskop geteilten Paarungshälften gebildet ist, wobei jede Hälfte ein Elementumschließungselement aufweist.

In der Schrift US 4964710 A1 wird ein starres Endoskop beschrieben, welches mit einem Objektivsystem, einer Okularlinse und einer Zwischenrelaislinse ausgestattet. Das Relaissystem ist ein Hybridsystem, das sowohl Kunststoff- als auch Glaselemente verwendet. Die Kunststoffelemente bestehen aus einer geradzahligen Anzahl (N) axial ausgerichteter Linsen, die jeweils eine Länge in der gleichen Größenordnung wie ihr Durchmesser aufweisen. Die Glaselemente sind eine ungeradzahlige Anzahl (N minus 1) von axial ausgerichteten Glas-Planzylindern, deren Stirnflächen poliert sind.

Die Schrift EP 1890173 A1 beschreibt eine Methode zur Herstellung eines Lichtleiters, wie er in derartigen Endoskopen einsetzbar ist. Dabei werden eine Vielzahl von optischen Fasern gebündelt und anschließend das Faserbündel an einem Teil eines Mundstücks geschnitten, das an einem Zwischenteil des Faserbündels befestigt ist. So wird das Faserbündel in ein erstes optisches Faserbündel und ein zweites optisches Faserbündel aufgeteilt. Die Teilungsflächen des ersten und zweiten optischen Faserbündels haben die gleichen Eigenschaften und Bedingungen, da die ersten und zweiten optischen Faserbündel aus dem Faserbündel gebildet sind, das durch Bündelung der gleichen optischen Fasern erhalten wird. Das erste optische Faserbündel ist in einem Einführungsabschnitt eines Endoskops montiert und das zweite optische Faserbündel ist in einem flexiblen Schlauch montiert, womit ein erster Lichtleiter in dem Einführungsabschnitt des Endoskops und ein zweiter Lichtleiter in dem flexiblen Schlauch ausgebildet sind. Dadurch entsteht eine trennbare Lichttransmissionsstrecke des Lichtleiters.

Da aufgrund ihrer Einmal-Verwendung derartige Endoskope unter einem hohen Kostendruck stehen, müssen die Baugruppen bzw. Komponenten kostenoptimiert herstellbar sein. Eine der Hauptkomponenten zur Bildgebung und Beleuchtung stellen Lichtleiter oder Bildleiter dar. Diese werden derzeit noch in vergleichsweise aufwendigen Prozessschritten montiert bzw. bearbeitet. Oft sind es komplexe mechanische Komponenten, teils kombiniert mit optischen Elementen, wie Linsen, die diese Licht- bzw. Bildleiter beinhalten, und teils sind es auch aufwendige Bearbeitungsschritte, wie das Schleifen und Polieren der Endfläche, die derzeitige Lichtleiter bzw. Bildleiter vergleichsweise kostenaufwendig machen.

Andererseits müssen aber auch noch gewisse lichttechnische Anforderungen beim endoskopischen Einsatz insbesondere in der Medizintechnik berücksichtigt werden. Dies sind neben möglichst verlustfreier Bereitstellung des von einer Lichtquelle bereitgestellten Lichtes an den Untersuchungsort, farbtreue bzw. gezielt farbige Darstellung des Untersuchungsortes auch die Vermeidung unnötige Wärme an den Untersuchungsort einzubringen.

Bei Verwendung von aktiven elektronischen Bauelementen, wie beispielsweise Kamera-Chips und/ oder LED zur Beleuchtung müssen zudem noch Anforderungen hinsichtlich elektrischer Isolation, elektrischer Abschirmung, sowie Patientenableitströmen berücksichtigt werden, die je nach Einsatzgebiet des Endoskops maximale Grenzwerte nicht überschreiten dürfen. So ist beispielsweise bei Anwendungen am Herzen ein max. Ableitstrom von 10 µA gefordert, was einer CF-Klassifizierung entspricht (vergl. EN 60601-1, 3. Ausgabe Tab. 3).

Neben diesen lichttechnischen und elektrischen Anforderungen sind auch noch Anforderungen hinsichtlich der Biokompatibilität zu beachten. Für die Biokompatibilität ist es erforderlich sicherzustellen, dass das Material mit menschlichem Organismus verträglich ist. Für Medizinprodukte, die in Kontakt zum menschlichen Körper kommen können, ist es regulatorisch gefordert, mögliche Interaktionen und unerwünschte Nebenwirkungen zu bestimmen und zu bewerten. Die Wahl der erforderlichen Prüfungen ergibt sich aus der Kontaktart und der Kontaktdauer im menschlichen Körper. Entsprechend der europäischen Richtlinie für Medizinprodukte MDD 93/42 EWG (kurz MDD) bzw. der Verordnung (EU) 2017/745 vom 5. April 2017 (kurz MDR) ist diese biologische Beurteilung eines Produktes immer dann notwendig, wenn ein unmittelbarer Kontakt von Werkstoff/Produkt mit dem Patienten besteht.

Die Hauptregelwerke zur biologischen Untersuchung und Beurteilung von Werkstoffen sind die DIN EN ISO 10993 und die Prüfung nach United States Pharmacopeia Class VI (USP Class VI). Obwohl die deutlich umfangreichere ISO 10993 ursprünglich den Test nach USP Class VI ersetzen sollte, wird die USP-Prüfung heute insbesondere sehr häufig zur Beurteilung von biokompatiblen Kunststoffen herangezogen. Dazu werden die für eine invasive Applikation vorgesehenen Materialien zum einen hinsichtlich ihrer chemischen Verbindung bewertet und zum anderen einem Zytotoxizitätstest unterzogen, bei dem möglichen toxischen Wirkungen auflebende Zellkulturen untersucht werden. Die Anforderungen hierfür sind in der DIN EN ISO 10993, insbesondere in den Teilen -1 und -5 zusammengefasst (DIN EN ISO 10993-1: 2010-04). In USA unterliegt dies den Anforderungen der FDA. Zur DIN EN ISO 10993 korrespondierende Anforderungen sind dort in der USP Class VI hinterlegt. Insbesondere ist auch die Biokompatibilität von Kunststoffen im Zusammenhang mit beispielsweise medizinischen und/oder therapeutischen Anwendungen in DIN EN ISO 10993 definiert.

Ferner ist die Biokompatibität im Zusammenhang mit Lebensmitteln, Futtermitteln und Bedarfsgegenständen relevant, wie sie beispielsweise in der BRD im LFGB geregelt sind sowie in entsprechenden europarechtlichen Normen.

Weiterhin kommt der Auslegung der Endoskope als Einweg-Endoskope zu Gute, dass die als Aufbereitungsmethoden bekannten Reinigungs-/ Desinfektionsverfahren mit stark basischen Lösungen und die Sterilisation mittels Autoklavieren bei Temperatur bis zu 135° C und typischen Dampfdrücken von etwa 3 bar bei der Materialauswahl nicht in diesem Maße berücksichtigt werden müssen, was insbesondere auch eine kostengünstigere Materialauswahl erlaubt. Lediglich sind RoHS- als auch REACH-Bestimmungen bei den Materialien zu berücksichtigen.

Die eigene Anmeldung der Anmelderin mit dem Aktenzeichen DE 10 2018 107 523 beschreibt Beleuchtungslichtleiter oder Bildleiter für Einweg-Endoskope bzw. Baugruppen mit Beleuchtungslichtleitern, Bildleitern und/ oder Kameras, die insbesondere kostengünstig in der Herstellung sind und andererseits typische lichttechnische Anforderungen für Endoskope in der Medizintechnik, insbesondere eine hohe Transmission und eine hohe Farbwiedergabetreue ermöglichen bei gleichzeitig hoher Biokompatibilität und geringer Zytotoxizität entsprechend der medizintechnischen Anforderungen und Wirkungen. Dies wird nach der eigenen Anmeldung der Anmelderin mit dem Aktenzeichen DE 10 2018 107 523 realisiert, indem die proximale und/oder die distale Endfläche des Beleuchtungslichtleiters und/oder des Bildleiters für Baugruppen umfassend einen Beleuchtungslichtleiter und/oder Bildleiter und/oder eine Kamera, wie beispielsweise ein Einweg-Endoskop, zumindest teil- oder abschnittsweise mindestens ein transparentes Kunststoffelement umfasst oder ein transparenter Kunststoff an die proximale und/oder die distale Endfläche angeformt ist. Der transparente Kunststoff ist biokompatibel und/ oder weist nicht toxische Eigenschaften auf menschliche oder tierische Zellkulturen für Einwirkzeiten kleiner einem Tag. Damit können sehr kostengünstig Beleuchtungslichtleiter oder Bildleiter hergestellt werden, bei denen eine ansonsten aufwendige Endenbearbeitung (welche auch Terminierung genannt wird), das heißt das Schleifen und Polieren der proximalen bzw. distalen Endfläche, entfallen kann. Die Biokompatibilität bzw. die nicht toxischen Eigenschaften des Kunststoffs erlaubt einen invasiven Eingriff im Körper (in vivo) oder ermöglicht In-Vitro-Untersuchungen an Zellkulturen und/oder Blutproben, ohne diese zu schädigen bzw. zu verändern. Durch die Wahl des Kunststoffes können hier optisch hochwertige Systeme bereitgestellt werden, die insb. den lichttechnischen Anforderungen für Endoskope gerecht werden, zumal die Temperaturbeständigkeit des Kunststoffes insbesondere für Einweg-Endoskope weniger hoch sein muss, was die Auswahl weniger einschränkt. Geeignete Kunststoffe sind Kunststoffe aus mindestens einer der Materialklassen Cyclo-Olefin-Co-Polymere, Polycarbonate, Polyethylen-Teraphthalaten, Perfluoralkoxy-Polymere, Polyvinylidenfluoride, Polymethylmethacrylate Polymethylmethacrylimide, Acryl- Styrol-Acrylnitril-Co-Polymere oder Raumtemperatur-vernetzende Silikon, heißvernetzende Flüssig-Silikone, Epoxid-Gießharze oder -Kleber, thermisch oder UV-vernetzende Acrylat-Gießharze, Polyurethan-Gießharze, Polyester-Gießharze oder aus Mischungen und/ oder Kombinationen derselben. Bei der Auswahl ist hier auf entsprechend biokompatible Varianten zu achten, die den eingangs erwähnten Norm-Anforderungen gerecht werden. Geeignet sind hier insbesondere thermoplastische Kunststoffe, die sich zum einen leicht spritzgießen lassen und transparent sind, beispielsweise PC, PMMA, COC etc., aber auch Kunststoffe, die als Gießharze applizierbar sind. Damit können entsprechend glatte Oberflächen mit sehr geringem Rauheitswert realisiert werden. Zudem sind o.g. Kunststoffe als biokompatible Version erhältlich.

Es hat sich gezeigt, dass ein besonderer Bedarf an sehr dünnen Lichtleitern besteht. Denn die Lichtleiter nach dem Stand der Technik lassen sich nur sehr schwierig in ein Diagnose-, Operations- und/ oder Therapiegerät zur Einbringung in den menschlichen oder tierischen Körper oder zur in-vitro Untersuchung von menschlichen oder tierischen Blutproben oder sonstigen Körperzellen, insbesondere ein Endoskop oder ein Einweg-Endoskop, einführen. Darüber hinaus ist nachteilig, dass ein großer Teil der Endfläche des Lichtleiters durch nicht-lichtleitende Teile des Lichtleiters, also insbesondere durch eine Umhüllung bzw. einen Mantel und/oder eine an insbesondere das distale Ende des Lichtleiters angebrachte Hülse verkleinert wird.

Die Aufgabe der Erfindung besteht daher darin, einen Lichtleiter und/oder ein Diagnose-, Operations- und/oder Therapiegerät bereitzustellen, welches die bisherigen Schwächen des Standes der Technik zumindest mildert.

Die vorliegende Offenbarung betrifft daher gemäß einem Aspekt ein Diagnose-, Operations- und/ oder Therapiegerät zur Einbringung in den menschlichen oder tierischen Körper oder zur in-vitro Untersuchung von menschlichen oder tierischen Blutproben oder sonstigen Körperzellen, insbesondere ein Endoskop oder ein Einweg-Endoskop, umfassend einen Lichtleichter umfassend mindestens zwei optische Fasern, wobei die mindestens zwei optischen Fasern ein Faserbündel bilden, und einen die Fasern und/oder das Faserbündel zumindest teil- oder abschnittsweise umgebenden Mantel, wobei der Lichtleiter eine maximale äußere laterale Abmessung seiner Querschnittsfläche, beispielsweise einen Außendurchmesser seiner Querschnittsfläche, aufweist, welcher maximal 500 µm, bevorzugt maximal 200 µm, besonders bevorzugt maximal 100 µm und ganz besonders bevorzugt maximal 50 µm größer ist als die maximale äußere laterale Abmessung, beispielsweise der Außendurchmesser, der Querschnittsfläche des Faserbündels, wobei der Lichtleiter ein proximales und ein distales Ende aufweist und zumindest eine terminierte Endfläche an mindestens einem Ende, vorzugsweise am distalen Ende, wobei die terminierte Endfläche eine maximale laterale äußere Abmessung aufweist, beispielsweise einen Außendurchmesser, welche höchstens so groß ist wie die maximale äußere laterale Abmessung, beispielsweise der Außendurchmesser, der Querschnittsfläche des Lichtleiters.

Unter einem die Faser und/oder das Faserbündel zumindest teil- oder abschnittsweise umgebenden Mantel wird hierbei verstanden, dass der Mantel auch gegebenenfalls äußere Oberflächen des Faserbündels frei lassen kann.

Ein weiterer Aspekt der vorliegenden Schrift betrifft einen Lichtleiter umfassend mindestens zwei optische Fasern, wobei die mindestens zwei optischen Fasern ein Faserbündel bilden, und einen die Fasern und/oder das Faserbündel zumindest teil- oder abschnittsweise umgebenden Mantel, wobei der Lichtleiter eine maximale äußere laterale Abmessung seiner Querschnittsfläche, beispielsweise einen Außendurchmesser seiner Querschnittsfläche, aufweist, welcher maximal 500 µm, bevorzugt maximal 200 µm, besonders bevorzugt maximal 100 µm und ganz besonders bevorzugt maximal 50 µm, größer ist als die maximale laterale Abmessung, beispielsweise der Außendurchmesser, der Querschnittsfläche des Faserbündels, wobei der Lichtleiter ein proximales und ein distales Ende aufweist und zumindest eine terminierte Endfläche an mindestens einem Ende, vorzugsweise am distalen Ende, wobei die terminierte Endfläche eine maximale laterale äußere Abmessung aufweist, beispielsweise einen Außendurchmesser, welche höchstens so groß ist wie die maximale äußere laterale Abmessung, beispielsweise der Außendurchmesser, der Querschnittsfläche des Lichtleiters.

Eine solche Ausgestaltung des Diagnose-, Operations- und/oder Therapiegeräts bzw. des Lichtleiters weist eine Reihe von Vorteilen auf.

Es hat sich nämlich gezeigt, dass mit den Lichtleitern des Standes der Technik Schwierigkeiten beim Einführen dieser Lichtleiter in ein Diagnose-, Operations- und/ oder Therapiegerät zur Einbringung in den menschlichen oder tierischen Körper oder zur in-vitro Untersuchung von menschlichen oder tierischen Blutproben oder sonstigen Körperzellen, wie beispielsweise ein Endoskop, bestehen. Denn die Fasern eines solchen Lichtleiters müssen gefasst sein, also zumindest an einem Ende, vorzugsweise am distalen Ende, so ausgebildet sein, dass es nicht zu einem Zerfasern des Bündels kommt, was ein Einführen in ein entsprechendes Diagnose-, Operations- und/oder Therapiegerät unmöglich machen würde. Daher weisen üblicherweise Lichtleiter an einem Ende, bevorzugt am distalen Ende, eine Hülse auf, die ein solches Zerfasern verhindert. Zumeist sind die Fasern bzw. ist das Faserbündel auch mit einer Hülle, beispielsweise einem Schrumpfschlauch, versehen.

Allerdings sind damit sehr feine Lichtleiter, also Lichtleiter, welche nur eine sehr geringe effektive äußere laterale Abmessung aufweisen, nicht herstellbar. Sowohl die Umhüllung des Faserbündels als auch die Hülse führen nämlich dazu, dass der resultierende Lichtleiter eine relativ große maximale äußere Abmessung, wie beispielsweise einen Außendurchmesser, aufweist, wobei diese maximale äußere Abmessung, beispielsweise der Außendurchmesser, des Lichtleiters auch zu einem wesentlichen Teil von der Umhüllung, beispielsweise also einem Schrumpfschlauch, und/oder von der Hülse beeinflusst wird.

Daher ist gemäß der vorliegenden Offenbarung der Lichtleiter derart ausgestaltet, dass er einen Mantel aufweist, welcher die vom Lichtleiter umfassten Fasern bzw. das vom Lichtleiter umfasste Faserbündel zumindest abschnittsweise umgibt, wobei der Lichtleiter eine maximale äußere laterale Abmessung - beispielsweise einen Außendurchmesser - aufweist, der maximal 500 µm, bevorzugt maximal 200 µm, besonders bevorzugt maximal 100 µm und ganz besonders bevorzugt maximal 50 µm größer ist als die maximale äußere laterale Abmessung, beispielsweise also der Außendurchmesser, des Faserbündels. Der Lichtleiter weist ein proximales und ein distales Ende auf sowie zumindest eine terminierte Endfläche an mindestens einem Ende, vorzugsweise am distalen Ende, wobei die terminierte Endfläche eine maximale äußere laterale Abmessung aufweist, beispielsweise einen Außendurchmesser, welche höchstens so groß ist wie die maximale äußere laterale Abmessung, also beispielsweise der Außendurchmesser, des Lichtleiters.

Mit anderen Worten sind also Mantel und/oder die terminierte Endfläche so ausgestaltet, dass sie die maximale äußere laterale Abmessung der Querschnittsfläche des Lichtleiters nur geringfügig vergrößern. Die maximale äußere laterale Abmessung des Lichtleiters ist insbesondere deshalb von besonderem Interesse, weil diese letztlich bestimmt, ob der Lichtleiter durch eine bestimmte Öffnung hindurchpasst und mithin geeignet ist, in ein Diagnose-, Operations- und/oder Therapiegerät eingeführt zu werden, oder nicht.

Sofern der Lichtleiter eine zumindest im Rahmen der Messgenauigkeit runde bzw. kreisförmige Querschnittsfläche aufweist, kann der Außendurchmesser des Lichtleiters beispielsweise diese maximale äußere laterale Abmessung der Querschnittsfläche sein.

Im Rahmen der vorliegenden Offenbarung gelten die folgenden Definitionen:
Eine terminierte Endfläche ist eine geschlossene Lichtaustrittsfläche. Insbesondere kann unter einer terminierten Endfläche eine Endfläche verstanden werden, welche eine optische Qualität aufweist oder an welcher nur geringe optische Verluste auftreten. Beispielsweise treten nur geringe optische Verluste auf, wenn die Endfläche eine geringe Oberflächenrauigkeit aufweist. Bevorzugt kann hier eine Oberflächenrauigkeit einer terminierten Endfläche, Rₐ, von höchstens 1,0 µm oder weniger, bevorzugt von 0,5 µm oder weniger und ganz besonders bevorzugt von 0,1 µm oder weniger, wobei eine praktisch erzielbare Untergrenze bei einer Oberflächenrauigkeit Rₐ von etwa 100 Ä liegt. Im weiteren Sinne kann also eine terminierte Endfläche auch als eine Endfläche mit definierten Endflächeneigenschaften verstanden werden, wie beispielsweise einer definierten Oberflächenrauigkeit.

Das proximale Ende des Lichtleiters bzw. des Faserbündels ist dasjenige Ende des Lichtleiters, welches einer Beleuchtungslichtquelle zugeordnet ist. Allgemein weist der Lichtleiter und/oder das Faserbündel ein proximales Ende, welches einer Lichtquelle zugeordnet ist, auf, sowie ein distales Ende, welches nicht der Lichtquelle, sondern dem zu beleuchtenden Ort zugeordnet ist auf.

Im Rahmen der vorliegenden Offenbarung werden die Begriffe der terminierten Endfläche und der Endfläche mit Terminierung weitgehend synonym verwendet. Dabei wird eine "Terminierung", sofern nicht ausdrücklich anders ausgeführt, nicht als ein Gegenstand verstanden, sondern beschreibt vielmehr den Zustand der Endfläche. Es ist allerdings allgemein möglich, dass eine Terminierung einer Endfläche durch ein separates Bauteil erfolgt.

Unter einem Lichtleiter wird im Rahmen der vorliegenden Offenbarung ein Bauteil verstanden, welches eingerichtet ist, elektromagnetische Strahlung, insbesondere elektromagnetische Strahlung im sichtbaren Spektralbereich, von einer Lichtquelle aus an einen anderen Ort zu leiten, sodass dieser andere Ort beleuchtet ist. Insbesondere kann daher unter einem Lichtleiter im Sinne der vorliegenden Offenbarung auch ein Beleuchtungslichtleiter verstanden werden.

Ein Faserbündel umfasst im Rahmen der vorliegenden Offenbarung mindestens zwei Fasern. Üblicherweise umfassen Faserbündel zwischen 100 und 1000 Fasern, je nach genauer Ausgestaltung des Faserbündels, wobei die genaue Zahl der von einem Faserbündel umfassten Fasern variiert in Abhängigkeit vom jeweiligen Faserdurchmesser, wobei hier typische Durchmesser zwischen 30 µm und 100 µm liegen, und/oder Faserbündeldurchmesser, wobei letztere insbesondere vom genauen Typ des Diagnose-, Operations- und/oder Therapiegeräts, wie einem Endoskop, abhängig sind. Im Rahmen der vorliegenden Offenbarung sind insbesondere dünne Lichtleiter und mithin auch entsprechend dünne Faserbündel von besonderem Interesse, bei denen die Faserbündel eher um die 100 Fasern umfassen oder wenige hundert Fasern.

Unter der lateralen Abmessung einer Fläche wird im Rahmen der vorliegenden Offenbarung eine Längenkenngröße der Fläche verstanden, beispielsweise die Länge und/oder die Breite einer Fläche. Ist die Fläche im Rahmen der Messgenauigkeit annähernd rund oder kreisförmig ausgebildet, kann beispielsweise die Fläche durch den Durchmesser beschrieben werden. Für ein Bauteil, wie beispielsweise einen Lichtleiter oder eine optische Faser, können dabei aufgrund des inneren Aufbaus mehrere laterale Abmessungen bedeutsam sein. Beispielsweise umfasst eine optische Faser einen Kern und ein sogenanntes Cladding, welcher um den Kern der Faser herum angeordnet ist. Ein Lichtleiter kann im Rahmen der vorliegenden Offenbarung so ausgestaltet sein, dass er ein Faserbündel und einen dieses Faserbündel umgebende Mantel umfasst. Der Lichtleiter kann daher durch wenigstens zwei laterale Abmessungen einer Schnittfläche, welche im Wesentlichen senkrecht zur Längsachse des Lichtleiters bzw. des vom Lichtleiter umfassten Faserbündels liegt, und auch als Querschnitt oder Querschnittsfläche bezeichnet werden kann, beschrieben werden: eine erste laterale Abmessung, welche sich auf das Faserbündel bezieht, und eine zweite laterale Abmessung, welche sich auf das Faserbündel und den dieses zumindest teil- oder abschnittsweise umgebenden Mantel bezieht. Da der Mantel um das Faserbündel herum angeordnet ist, kann diese zweite laterale Abmessung auch als äußere laterale Abmessung bezeichnet werden. Ist der Lichtleiter annähernd rund ausgebildet, wird diese äußere laterale Abmessung auch als Außendurchmesser bezeichnet. Für den Fall, dass die Querschnittsfläche des Lichtleiters annähernd rund oder kreisförmig oder kreisabschnittsförmig (beispielsweise als Halbkreis) ausgebildet ist, kann der Außendurchmesser auch als maximale äußere laterale Abmessung verstanden werden. Für den Fall einer nicht runden oder zumindest kreisabschnittsförmigen Ausgestaltung, beispielsweise für den Fall einer rechteckförmigen oder zumindest annähernd rechteckförmigen Ausgestaltung, der Querschnittsfläche erfolgt im Rahmen der vorliegenden Offenbarung die Charakterisierung der Querschnittsfläche durch die maximale äußere laterale Abmessung, welche für den Fall eines Rechtecks die Diagonale sein kann.

Sofern im Rahmen der vorliegenden Offenbarung auf die Querschnittsfläche des Lichtleiters und/oder des Faserbündels abgestellt wird, handelt es sich dabei, sofern dies nicht ausdrücklich anders ausgeführt ist, um die Fläche, welche bei einem Schnitt durch das Faserbündel bzw. den Lichtleiter erhalten wird, wobei dieser Schnitt senkrecht oder im Wesentlichen senkrecht zur Längsachse des Faserbündels bzw. des Lichtleiters liegt. Im Wesentlichen senkrecht bedeutet hierbei, dass der Normalenvektor der Querschnittsfläche und die Längsachse des Faserbündels bzw. des Lichtleiters voneinander um einen Winkel von nicht mehr als 5° abweichen.

Unter einem Mantel wird im Rahmen der vorliegenden Offenbarung ein Material oder ein Erzeugnis verstanden, welche das Faserbündel und/oder die das Faserbündel bildenden Fasern zumindest abschnittsweise umgibt. Der Mantel kann dabei zunächst beispielsweise als separates Formteil, also beispielsweise als Schlauch und/oder Folie, ausgebildet sein. Dies ist jedoch nicht erforderlich, sondern es ist vielmehr möglich, dass der Mantel erst in situ ausgebildet wird, beispielsweise, indem ein fluides Material das Faserbündel umhüllt und/oder imprägniert und anschließend aushärtet und so den Mantel bildet.

Eine solche Ausgestaltung des Diagnose-, Operations- und/oder Therapiegeräts bzw. des Lichtleiters nach der vorliegenden Anmeldung weist eine Reihe von Vorteilen auf. Insbesondere ist der Lichtleiter auf diese Weise sehr dünn und flexibel ausgebildet. Unter Verwendung eines besonders dünnen Mantels für den Lichtleiter dient insbesondere auch nur ein geringer Anteil der Endfläche, sofern überhaupt, nicht der Ausleuchtung, sodass eine besonders hohe Lichtintensität vorliegt. Mit anderen Worten bedeutet dies: Durch den dünnen Mantel ergibt sich für den so ausgestalteten Lichtleiter eine maximal mögliche Fläche an lichtübertragenden Fasern in der Querschnittsfläche bzw. Endfläche. Dies bedeutet also einen maximalen Lichttransport in Lumen und führt entsprechend zu einer maximalen Lichtausbeute bezogen auf zur Verfügung stehendem Querschnitt für Lichtleiter nach der vorliegenden Offenbarung im Vergleich mit den Lichtleitern nach dem Stand der Technik.

Gemäß einer Ausführungsform des Diagnose-, Operations- und/oder Therapiegeräts bzw. des Lichtleiters umfasst der Mantel des Lichtleiters Kunststoff, bevorzugt einen biokompatiblen Kunststoff, insbesondere einen biokompatiblen Kunststoff gemäß DIN EN ISO 10993 und/oder einen biokompatiblen Kunststoff für Anwendungen im Lebensmitteln, insbesondere im Sinne des LFGB und/oder entsprechender internationalen und/oder europarechtlicher Normen, und/ oder einen Kunststoff, der nicht toxische Eigenschaften auf menschliche oder tierische Zellkulturen für Einwirkzeiten kleiner einem Tag aufweist.

Unter Kunststoff wird im Rahmen der vorliegenden Offenbarung ein Material verstanden, welches Makromoleküle umfasst oder überwiegend, also zu mindestens 50 Gew.-%, oder im Wesentlichen, also zu mindestens 90 Gew.-%, oder sogar vollständig aus Makromolekülen besteht. Als ein Makromolekül wird im Rahmen der vorliegenden Offenbarung ein Molekül verstanden, welches eine Molmasse von mindestens 10000 g/mol aufweist. Im Rahmen der vorliegenden Offenbarung wird der Begriff des Kunststoffs, sofern nicht ausdrücklich anders ausgeführt, als synonym mit dem Begriff des Polymers verwendet.

Eine solche Ausgestaltung des Lichtleiters ist vorteilhaft, weil Kunststoffe in vielfältigen Varianten vorliegen und entsprechend den Anforderungen an den Mantel so ausgestaltet sein können, dass die notwendigen Anforderungen an den Leichtleiter, beispielsweise hinsichtlich dessen Flexibilität und/oder Starrheit, der Temperatur- und/oder Klima- und/oder Feuchtebeständigkeit sowie der optischen Eigenschaften angepasst werden kann. Aufgrund der hohen Flexibilität bei der Auswahl eines Kunststoffes ist es dabei beispielsweise auch möglich, einen biokompatiblen Kunststoff und/oder einen Kunststoff, der nicht toxische Eigenschaften auf menschliche oder tierische Zellkulturen für Einwirkzeiten kleiner einem Tag aufweist, und/oder einen sterisilisierbaren, vorzugsweise einen mit Ethylenoxid sterilisierbaren, Kunststoff zu wählen.

Unter einem biokompatiblem Material wird im Rahmen der vorliegenden Offenbarung ein Material verstanden, das mit dem menschlichen Organismus verträglich ist. Insbesondere kann darunter ein biokompatibles Material im Sinne der DIN EN ISO 10993 und/oder für Lebensmittel, Bedarfsgegenstände und/oder Futtermittel im Sinne des LFGB und/oder entsprechenden internationalen und/oder europarechtliche Normen verstanden werden.
Wie vorstehend bereits ausgeführt, ist es für Medizinprodukte, die in Kontakt zum menschlichen Körper kommen können, regulatorisch gefordert, mögliche Interaktionen und unerwünschte Nebenwirkungen zu bestimmen und zu bewerten, wobei sich die Wahl der erforderlichen Prüfungen aus der Kontaktart und der Kontaktdauer im menschlichen Körper ergibt. Entsprechend der europäischen Richtlinie für Medizinprodukte MDD 93/42 EWG bzw. der Verordnung (EU) 2017/745 (MDR) ist diese biologische Beurteilung eines Produktes immer dann notwendig, wenn ein unmittelbarer Kontakt von Werkstoff/Produkt mit dem Patienten besteht.

Die Hauptregelwerke zur biologischen Untersuchung und Beurteilung von Werkstoffen sind die DIN EN ISO 10993 und die Prüfung nach United States Pharmacopeia Class VI (USP Class VI). Obwohl die deutlich umfangreichere ISO 10993 ursprünglich den Test nach USP Class VI ersetzen sollte, wird die USP-Prüfung heute insbesondere sehr häufig zur Beurteilung von biokompatiblen Kunststoffen herangezogen. Dazu werden die für eine invasive Applikation vorgesehenen Materialien zum einen hinsichtlich ihrer chemischen Verbindung bewertet und zum anderen einem Zytotoxizitätstest unterzogen, bei dem möglichen toxischen Wirkungen auflebende Zellkulturen untersucht werden. Die Anforderungen hierfür sind in der DIN EN ISO 10993, insbesondere in den Teilen -1 und -5 zusammengefasst (DIN EN ISO 10993-1: 2010-04). In USA unterliegt dies den Anforderungen der FDA. Zur DIN EN ISO 10993 korrespondierende Anforderungen sind dort in der USP Class VI hinterlegt.

Vorzugsweise ist der Kunststoff ausgewählt aus der Gruppe der Cyclo-Olefin-Co-Polymere, Polycarbonate, Polyethylen-Teraphthalaten, Perfluoralkoxy-Polymere, Polyvinylidenfluoride, Polymethylmethacrylate, Polymethylmethacrylimide, Acryl Styrol-Acrylnitril-Co-Polymere oder Raumtemperatur-vernetzende Silikon, heißvernetzende Flüssig-Silikone, Epoxid-Gießharze oder -Kleber, thermisch oder UV-vernetzende Acrylat-Gießharze, Polyurethan-Gießharze, Polyester-Gießharze oder aus Mischungen und/ oder Kombinationen derselben. Insbesondere sind hier auch kombinierte Vernetzungsprozesse denkbar, beispielsweise eine kombinierte UV- und Heißvernetzung.

Diese Materialien sind bevorzugt, denn dabei handelt es sich um leicht verarbeitbare, gängige Kunststoffe, welche auch in sehr guter Qualität kostengünstig herstellbar sind. Damit lässt sich auf einfache Art ein Mantel herstellen, welcher eine gute mechanische Stabilität aufweist, sodass gleichzeitig das Faserbündel geschützt ist gegen mechanische Belastungen, wie beispielsweise ein Durchbiegen, aber auch eine ausreichende chemische Beständigkeit aufweist.

Gemäß einer weiteren Ausführungsform des Diagnose-, Operations- und/oder Therapiegeräts und/oder des Lichtleiters umfasst der Mantel des Faserbündels einen Schrumpfschlauch, eine Folie, einen extrudierten Mantel und/ oder ein Material mit niederviskosen Fließeigenschaften, welches durch Sprühen, Tauchen, Gießen und/ oder Extrusion im Wesentlichen ohne Luftspalt direkt auf das Faserbündel aufbringbar ist, wobei jeweils das vom Mantel umfasste Material transparent, transluzent, opak und/oder eingefärbt vorliegen kann. Unter einer Ausgestaltung, dass der Mantel durch Extrusion im Wesentlichen ohne Luftspalt direkt auf das Faserbündel aufbringbar ist, wird im Rahmen der vorliegenden Offenbarung verstanden, dass ein möglicherweise vorhandener Spalt zwischen dem Mantel bzw. dem Mantelmaterial und dem Faserbündel nicht mehr als 10 µm beträgt, vorzugsweise deutlich weniger; wobei besonders bevorzugt kein Luftspalt gebildet ist.

Insbesondere eine Extrusion im Wesentlichen ohne Luftspalt wurde bislang nicht verwirklicht. Bei einer sogenannten Extrusion ohne Luftspalt, welche beispielsweise in der Offenlegungsschrift DE 10 2011 114 575 A1 beschrieben ist, kann es bedeutend leichter zu einem Bruch des Faserbündels kommen. Bei einer sogenannten Extrusion mit Luftspalt handelt es sich um eine Extrusion, bei der eine Verbindung, insbesondere eine materialschlüssige Verbindung, zwischen dem Faserbündel und dem Mantel nicht vorliegt. Mit anderen Worten sind dort der Mantel und das Faserbündel mechanisch entkoppelt.

Dies ist jedoch gemäß der Ausführungsform des Diagnose-, Operations- und/oder Therapiegeräts und/oder des Lichtleiters, bei welchem der Mantel direkt auf das Faserbündel aufbringbar ist, nicht mehr der Fall. Vielmehr besteht nunmehr ein enger, inniger Kontakt zwischen dem Material des Mantels und dem Material des Faserbündels bzw. zwischen dem Material des Mantels und wenigstens einer Faser des Faserbündels. Überraschenderweise hat sich gezeigt, dass auf diese Weise anders als erwartet doch keine zu geringe mechanische Festigkeit des Lichtleiters resultiert. Insbesondere kommt es nicht zwangsläufig zu einem leichteren Bruch des Lichtleiters, wenn beispielsweise der Mantel extrem dünn, wie zuvor beschrieben, ausgeführt ist und/ oder wenn besonders weiche (mit niedriger Shore-Härte, beispielsweise ≤ 50 Shore A) und damit auch flexible Polymere in ihrem ausgehärteten bzw. endvernetzten Zustand zum Einsatz kommen. Die Ausführung eines Lichtleiters in der Form, dass ein enger Kontakt zwischen dem Mantel und dem Faserbündel bzw. zwischen dem Mantel und mindestens einer Faser des Faserbündels vorliegt, ist vorteilhaft, weil auf diese Weise eine leichte und wenig aufwendige Herstellung des Lichtleiters ermöglichst wird. Insbesondere ist es nicht mehr notwendig, beispielsweise beim Extrudieren eines Mantels ein Fluid zwischen Mantel und der Oberfläche des Faserbündels entlangströmen zu lassen, um einen Spalt zwischen Mantel und der Oberfläche des Faserbündels zu bilden und dadurch Mantel und Faserbündel mechanisch zu entkoppeln.

Vorzugsweise ist dabei der das Faserbündel und/oder die Fasern umgebende Mantel mittels Extrusion hergestellt und die Wandstärke des Materials ist maximal 0,15 mm, bevorzugt 0,1 mm und besonders bevorzugt maximal 0,05 mm dick, wobei das Mantelmaterial ein Thermoplast, insbesondere ein Fluor-Thermoplast, und/oder ein Hochtemperatur-Kunststoff, bevorzugt ein bei der jeweiligen Verarbeitungstemperatur niedrigviskoser Hochtemperatur-Kunststoff, ist oder umfasst, und/oder ein Compound umfassend einen solchen Thermoplasten oder einen Hochtemperatur-Kunststoff.

Geeignete Kunststoffmaterialien, insbesondere auch Bestandteile von Compounds, können dabei insbesondere sein:

| **Kunststoffklassen neu / alt** | **Beschreibung** | **Beispielhafte Handelsnamen** |
|---|---|---|
| TPE-A oder TPA | Thermoplastische Copolyamide | PEBAX® |
| TPE-E oder TPC | Thermoplastische Polyesterelastomere / Thermoplastische Copolyester | Hytrel® |
| TPE-O oder TPO | Thermoplastische Elastomere auf Olefinbasis, vorwiegend PP/EPDM | Dytron® |
| TPE-U oder TPU | Thermoplastische Elastomere auf Urethan basis | Elastollan® |
| TPE-V oder TPV | Thermoplastische Vulkanisate oder vernetzte thermoplastische Elastomere auf Olefinbasis, vorwiegend PP/EPDM | Santoprene® |
| PVC | Polyvinylchlorid | Troilit® |

Darüber hinaus kann insbesondere eine Ausgestaltung des Diagnose-, Operations- und/oder Therapiegeräts bzw. des Lichtleiters in der Form, dass der Mantel einen transparenten Kunststoff umfasst, vorteilhaft sein. Beispielsweise kann der Kunststoff gemäß dieser Ausgestaltung auch ein optischer Kunststoff sein, also ein Kunststoff, welcher beispielsweise in optischen Anwendungen verwendet wird, wie für Brillengläser und/oder optische Linsen. Dies ist insbesondere deshalb vorteilhaft, weil auf diese Weise beispielsweise eine Ausgestaltung des Diagnose-, Operations- und/oder Therapiegeräts bzw. des Lichtleiters möglich ist, bei welcher das Faserbündel und/oder die das Faserbündel bildenden Fasern zumindest abschnittsweise in das Mantelmaterial eingebettet und/oder von diesem zumindest abschnittsweise und/oder zumindest teilweise umgeben vorliegen können und es weiterhin auch möglich ist, die Terminierung der Endfläche so auszuführen, dass diese einstückig mit dem und durch den Mantel gebildet wird. Denn dadurch, dass gemäß einer Ausführungsform die Fasern und/oder das Faserbündel zumindest abschnittsweise in das Mantelmaterial eingebettet und/oder von diesem zumindest abschnittsweise und/oder zumindest teilweise umgeben vorliegen können, können in dem Bereichen des Lichtleiters, in denen der Mantel das Faserbündel und/oder die Faser zumindest teilweise umgibt, Endflächen erhalten werden, beispielsweise durch Schneiden, bei denen der Mantel die Fasern so fasst, dass eine Terminierung der Endfläche durch eine Hülse und/oder ein sonstiges separates, mit den Fasern und/oder dem Faserbündel nicht materialschlüssig verbundenes Bauteil, nicht mehr notwendig ist. Dabei kann es beispielsweise allgemein vorgesehen sein, dass das Mantelmaterial als Vergussmasse ausgebildet ist, welche ausgehend von der Endfläche zumindest wenige Millimeter, maximal zehn Millimeter, in das Faserbündel eindringt. Auf diese Weise ist es besonders einfach möglich, die Terminierung der Endfläche so auszuführen, dass diese einstückig mit dem und durch den Mantel gebildet wird. Auf diese Weise können sogar Endflächen erhalten werden, welche nicht durch ein Schneiden des Faserbündels erfolgen, sondern durch die Oberflächenspannung des Mantelmaterials selbst als terminierte Endfläche in ausreichender Qualität vorliegen.

Gemäß einer weiteren Ausführungsform des Diagnose-, Operations- und/oder Therapiegeräts bzw. des Lichtleiters umfasst das Faserbündel eine Glasfaser und/oder eine polymerische optische Faser (POF). Insbesondere ist von dieser Ausführungsform umfasst der Fall, dass Faserbündel ein Glasfaserbündel oder ein POF-Faserbündel ist.

Unter einer Faser wird im Rahmen der vorliegenden Offenbarung ein Körper verstanden, dessen größte laterale Abmessung in einer Raumrichtung eines kartesischen Koordinatensystems mindestens um den Faktor 10, vorzugsweise mindestens um den Faktor 50, größer ist als in den beiden anderen, zu dieser ersten Raumrichtung senkrechten Raumrichtungen. Mit anderen Worten ist eine Faser ein sehr langer, dünner Körper.

Eine Glasfaser umfasst Glas. Die Glasfaser kann weiterhin neben dem glasigen Material noch ein weiteres Material umfassen, welches die Oberfläche des glasigen Materials zumindest teilweise umgibt, die sogenannte Schlichte. Je nach Einsatzzweck können unterschiedliche glasige Materialien für eine Glasfaser verwendet werden. Insbesondere kann die Glasfaser ein einkomponentiges und/oder ein mehrkomponentiges Glas umfassen. Beispielsweise kann die Glasfaser als im Wesentlichen einkomponentiges Glas Quarzglas umfassen und/oder insbesondere als Quarzglasfaser ausgebildet sein, wobei das Quarzglas auch dotiert vorliegen kann, beispielsweise mit OH-Ionen und/oder mit Fluor dotiert, und /oder als bspw. wasserreiche oder wasserarme Quarzglasvarianten vorliegt, wobei in diesem Fall noch immer von einem einkomponentigen Glas gesprochen wird, oder ein mehrkomponentiges Glas umfassen, beispielsweise ein mehrkomponentiges, silikatisches Glas. Weiterhin kann das Glas auch als Chalkogenidglas ausgebildet sein. Unter einer Quarzglasfaser oder Quarzfaser wird dabei auch eine Faser umfassend dotiertes Quarzglas verstanden.

Bevorzugt umfasst die optische Faser einen Faserkern und einen Faserrand bzw. eine Fasermantelschicht. In bevorzugten Ausführungsformen besteht die Kernschicht aus einem Kernglas. In anderen Ausführungsformen besteht der Faserkern aus einem Kernpolymer. Geeignete Kernpolymere sind beispielsweise Polyacrylat, Polymethacrylat, Polyurethan, Polyester, Polyamid, und Mischungen daraus.

Bevorzugt umfasst die optische Faser Fasermantel, welche den Faserkern umgibt. In bevorzugten Ausführungsformen umfasst der Fasermantel ein Mantelglas. In anderen Ausführungsformen umfasst der Fasermantel ein Mantelpolymer. Geeignete Mantelpolymere sind beispielsweise Polyacrylat, Polymethacrylat, Polyurethan, Polyester, Polyamid und Mischungen daraus.

Bevorzugt weist der Fasermantel einen Gehalt an Halogenen bzw. Halogeniden von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist der Fasermantel frei von Halogen. Halogene sind beispielsweise Chlor, Fluor, Brom und/oder lod bzw. deren Anionen. Eine zu hohe Konzentration an Halogenen im Fasermantel führt zur Bildung der entsprechenden Halogensäuren, insbesondere beispielsweise bei der Dampfsterilisation. Die entsprechenden Halogensäuren können die Beständigkeit des optischen Faserartikels mindern sowie aus diesem austreten. Insbesondere greifen die Halogensäuren Materialien wie beispielsweise Edelstahl von Autoklaven und Endoskopen an und führen zur Bildung von unerwünschtem Rost.

Bevorzugt weist der Faserkern einen Gehalt an Halogenen bzw. Halogeniden von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist die Kernschicht frei von Halogen. Erfindungsgemäße Halogene sind beispielsweise Chlor, Fluor, Brom und/oder Iod bzw. deren Anionen. Eine zu hohe Konzentration an Halogenen im Faserkern führt zur Bildung der entsprechenden Halogensäuren, insbesondere beispielsweise bei der Dampfsterilisation. Die entsprechenden Halogensäuren können die Beständigkeit des optischen Faserartikels mindern sowie aus diesem austreten. Insbesondere greifen die Halogensäuren Materialien wie beispielsweise Edelstahl von Autoklaven und Endoskopen an und führen zur Bildung von unerwünschtem Rost.

In bestimmten Ausführungsformen ist die optische Faser eine Quarzfaser. In einer bestimmten Ausführungsform weist der Fasermantel und/oder weist der Faserkern einen Anteil von mindestens 76 Gew.-% Quarz auf, weiter bevorzugt mindestens 81 Gew.-%, weiter bevorzugt mindestens 84 Gew.-%, weiter bevorzugt mindestens 88 Gew.-%, weiter bevorzugt mindestens 92 Gew.-%, weiter bevorzugt mindestens 95 Gew.-%, weiter bevorzugt mindestens 97 Gew.-%, weiter bevorzugt mindestens 98 Gew.-%. Ein höherer Quarzanteil führt zu einer erhöhten chemischen Beständigkeit sowie zu einer erhöhten Temperaturbeständigkeit.

In einer bestimmten Ausführungsform weist das Kernglas folgende Merkmale auf:
Bevorzugt umfasst das Kernglas wenigstens 8 Gew.-%, weiter bevorzugt wenigstens 23 Gew.-%, mehr bevorzugt wenigstens 24 Gew.-% und besonders bevorzugt wenigstens 25 Gew.-% oder sogar wenigstens 26 Gew.-% SiO₂. In einer besonderen Ausführungsform kann das Kernglas sogar mindestens 28,3 Gew.-% SiO₂ umfassen, ganz besonders bevorzugt mindestens 34 Gew.-% SiO₂. In einigen bevorzugten Ausführungsformen umfasst das Kernglas sogar wenigstens 35 Gew.-% SiO₂, weiter bevorzugt wenigstens 42 Gew.-%.

Bevorzugte Kerngläser dieser Erfindungen umfassen die folgenden Komponenten in den folgenden Zusammensetzungsbereichen in Gewichtsprozent:

| **Komponente** | **von** | **bis** |
|---|---|---|
| B₂O₃ | 0 | 24 |
| SiO₂ | 23 | 62,1 |
| Al₂O₃ | 0 | 10 |
| Li₂O | 0 | 10 |
| Na₂O | 0 | 18,5 |
| K₂O | 0 | 25,7 |
| BaO | 0 | 57,8 |
| ZnO | 0 | 40 |
| La₂O₃ | 0 | 25 |
| ZrO₂ | 0 | 10 |
| HfO₂ | 0 | 14,2 |
| SnO₂ | >0 | 2 |
| MgO | 0 | 8 |
| CaO | 0 | 8 |
| SrO | 0 | 24,4 |
| Ta₂O₅ | 0 | 22 |
| Y₂O₃ | 0 | 11,9 |
| Rb₂O | 0 | 15 |
| Cs₂O | 0 | 21 |
| GeO₂ | 0 | 7,5 |
| F | 0 | 2 |
| Σ R₂O | 5 | 20 |
| Σ MgO, CaO, SrO, ZnO | 20 | 42 |

R₂O ist jeweils die Summe der Gehalte aller Alkalimetalloxide.

Eine oder mehrere der folgenden Komponenten kann vom Kernglas umfasst sein: Cs₂O, Rb₂O, MgO, CaO, SrO, Gd₂O₃, Lu₂O₃, Sc₂O₃, Y₂O₃, In₂O₃, Ga₂O₃ und WO₃.

Folgende Komponenten sollten im Kernglas bevorzugt nicht enthalten sein oder lediglich in Konzentrationen von jeweils höchstens 500 ppm, die durch unvermeidbare Verunreinigungen der Rohstoffe bedingt sind: TiO₂, CeO₂, Nb₂O₅, MoO₃, Bi₂O₃, PbO, CdO, Tl₂O, As₂O₃, Sb₂O₃, SO₃, SeO₂, TeO₂, BeO, radioaktive Elemente und färbende Komponenten, soweit im Text nicht anders beschrieben. Insbesondere auf TiO₂ sollte verzichtet werden, weil diese Komponente für eine ausgeprägte Absorption im UV-Bereich führen kann. In bevorzugten Ausführungsformen wird auch auf die Komponente WO₃ verzichtet.

Die Komponenten TiO₂, CeO₂, Nb₂O₅ und/oder Bi₂O₃ können bis maximal 0,5 Gew.-%, bevorzugt bis 0,3 Gew.-% und besonders bevorzugt bis 0,2 Gew.-% im Kernglas enthalten sein. In einer bevorzugte Ausführungsform ist das Kernglas frei von diesen Komponenten.

Bevorzugt ist das Kernglas frei von optisch aktiven Komponenten, insbesondere Sm₂O₃, Nd₂O₃, Dy₂O₃, Pr₂O₃, Eu₂O₃, Yb₂O₃, Tb₂O₃, Er₂O₃, Tm₂O₃ und/oder Ho₂O₃. CeO₂ absorbiert im UV-Bereich, so dass bevorzugte Kerngläser kein CeO₂ enthalten.

Der Gehalt der Komponenten Erdalkalimetalloxide, La₂O₃, Ta₂O₅, ZrO₂ und HfO₂ beträgt in Summe vorzugsweise und insbesondere für Kerngläser mit Brechwerten von mehr als 1,65 wenigstens 40 Gew.-%, weiter bevorzugt wenigstens 42 Gew.-%, mehr bevorzugt wenigstens 50 Gew.-% und besonders bevorzugt wenigstens 55 Gew.-%. Wenn der Gehalt dieser Komponenten zu niedrig ist, kann der bevorzugte Brechungsindex normalerweise nicht erreicht werden. Formulierungsbedingt sollte diese Summe einen Wert von 72 Gew.-% nicht übersteigen.

In einer bestimmten Ausführungsform weist das Mantelglas folgende Merkmale auf:
Bevorzugt weist das Mantelglas einen Gehalt an SiO₂ von >60 Gew.-%, weiter bevorzugt >65 Gew.-% und besonders bevorzugt von mindestens 69 Gew.-% auf. Der SiO₂-Gehalt beträgt bevorzugt höchstens 75 Gew.-% und besonders bevorzugt bis zu 73 Gew.-%. Das Mantelglas ist tendenziell stärkeren Umwelteinflüssen ausgesetzt als das Kernglas. Ein hoher SiO₂-Gehalt verleiht bessere chemische Resistenz. Folglich ist der Gehalt an dieser Komponente im Mantelglas bevorzugt größer als im Kernglas.

Vorzugsweise wird die Zusammensetzung des Mantelglases so ausgewählt bzw. an die des Kernglases angepasst, dass der lineare thermische Ausdehnungskoeffizient des Mantelglases und derjenige des Kernglases sich möglichst wenig unterscheiden. Allgemein kann der thermische Ausdehnungskoeffizient (CTE) in einem Temperaturbereich von 20 bis 300°C für Faserkern und Fasermantel gleich oder unterschiedlich sein. Insbesondere ist der CTE unterschiedlich. Vorzugsweise ist der CTE des Mantels kleiner als der CTE des Faserkerns, typischerweise ist er mindestens um 1,0*10⁻⁶ /K kleiner, kann aber auch, je nach Glas typischerweise mindestens um 2,5^{∗}10⁻⁶ /K. kleiner sein. Der Faserkern weist typischerweise einen CTE von 6,5^{∗}10⁻⁶ bis 10^{∗}10⁻⁶/K auf, der Mantel einen CTE von 4,5^{∗}10⁻⁶ bis 6^{∗}10⁻⁶/K. Dadurch wird erreicht, dass der Kern der Faser beim Abkühlen stärker als der Fasermantel schrumpft, wodurch im Fasermantel eine die Faser schützende Druckspannung aufgebaut wird, was für die mechanische Belastbarkeit der Faser, insbesondere ihrer Biegefestigkeit zuträglich ist.

Die folgende Tabelle zeigt einige bevorzugte Zusammensetzungen von Mantelgläsern, die zusammen mit den Kerngläsern verwendet werden können. Die Mantelgläser umfassen (in Gew.-% auf Oxidbasis):

| **Oxide** | **Gruppe 1** | **Gruppe 2** | **Gruppe 3** | **Gruppe 4** |
|---|---|---|---|---|
| SiO₂ | 70 - 78 | 63 - 75 | 75 - 85 | 62 - 70 |
| Al₂O₃ | 5 - 10 | 1 - 7 | 1 - 5 | 1 - 10 |
| B₂O₃ | 5 - 14 | 0 - 3 | 10 - 14 | > 15 |
| Li₂O | frei | 0 - 1 | 0 - 3 | < 0,1 |
| Na₂O | 0 - 10 | 8 - 20 | 2 - 8 | 0 - 10 |
| K₂O | 0 - 10 | 0 - 6 | 0 - 1 | 0 - 10 |
| MgO | 0 - 1 | 0 - 5 | frei | 0 - 5 |
| CaO | 0 - 2 | 1 - 9 | frei | 0 - 5 |
| SrO | 0 - 1 | frei | frei | 0 - 5 |
| BaO | 0 - 1 | 0 - 5 | frei | 0 - 5 |
| Halogen | frei | frei | frei | frei |

In einer anderen bestimmten Ausführungsform ist das Kernglas und/oder das Mantelglas ein Chalcogenidglas, welches insbesondere Anwendungen im Infrarot-Bereich ermöglicht. Die folgende Tabelle zeigt bevorzugte Zusammensetzungen von Kernchalcogenidgläsern und/oder Mantelchalcogenidgläsern in Molprozent:

| **Komponente** | **Mol-%** |
|---|---|
| S | 50-90 |
| Ga | 0-25 |
| As | 0-40 |
| Ge | 0-35 |
| R¹ (zugegeben in Form von R¹Hal) | 0-7,25 |
| R² (zugegeben in Form von R²Hal) | 0-13,5 |
| M¹ (zugegeben in Form von M¹Hal₂) | 0-5 |
| M² (zugegeben in Form von M²Hal₂) | 0-7,25 |
| Ln (zugegeben in Form von LnHal₃) | 0-4 |
| Sum of Ga, As, and Ge | 10-42 |
| Sum of R¹, R², M¹, M², and Ln | 0-16 |
| Sum of Hal | 0-16 |

Hierbei ist Hal = Fluor, Chlor, Brom, und/oder Iod; Hal2 und/oder Hal3 = Chlor und/oder Brom; R¹ = Li, Na, K, Rb, und/oder Cs; R² = Ag und/oder Cu; M¹ = Mg, Ca, Sr, und/oder Ba; M² = Zn, Cd, Hg, und/oder Pb; Ln = La, Ce, Pr, Nd, Pm, Sm Eu, Gd, Tb, Dy, Ho, Er, Tm, Ty, Lu, Y, und Sc.

Besonders vorteilhaft ist es, wenn die Glasfasern, Faserstäbe oder gepressten Faserstäbe aus einem Pb- bzw. Schwermetall-freien Kernglas und Mantelglas bestehen. Derartige Fasersysteme bieten insbesondere eine hohe Transmission im VIS-Spektralbereich und zeigen aufgrund der vergleichsweisen hohen Transmission im blauen Spektralbereich eine hohe Farbtreue, was insbesondere wichtig ist bei der medizinischen Beurteilung von Gewebe. Oft entscheiden hier nur geringfügige Farbunterschiede des Gewebes, ob es sich um eine gutartige oder bösartige Gewebeveränderung handelt. Daher kommt es auf einen hohen CRI-Wert des Gesamtsystems aus Lichtquelle, Lichtleiter und bildgebender Einrichtung an, wobei CRI (Color Rendering Index) eine Kennzahl einer photometrischen Größe ist, mit der die Qualität der Farbwiedergabe von Lichtquellen gleicher korrelierter Farbtemperatur beschrieben wird. Ein CRI-Wert von > 90 kann mit den oben beschriebenen Glasfasern, Faserstäbe oder gepressten Faserstäbe erzielt werden. Derartige Fasersysteme sind seitens der Anmelderin unter dem Namen SCHOTT PURAVIS® bekannt und sind hinsichtlich ihrer Zusammensetzungen in der DE 102012100233 B4 und DE 102013208838 B4 beschrieben. Ähnliche Fasersysteme sind auch in der EP 2072477 B1 beschrieben, welche ebenfalls Pb-frei sind.

Insbesondere für die Verwendung in Endoskopen ist es von Vorteil, wenn Glasfasern, Faserstäbe oder gepressten Faserstäbe aus einem Glassystem bestehen, welches für das zu leitende Licht einen Akzeptanzwinkel 2α von größer 80°, besonders bevorzugt von größer 100° aufweist, was einer numerischen Apertur (NA) von größer 0,64, besonders bevorzugt größer 0,77 entspricht. Zum einen kann erreicht werden, dass insbesondere Licht von LEDs, welche i.d.R. einen sehr breiten Abstrahlwinkel aufweisen, ohne komplexe Optiken am proximalen Ende in die Glasfasern bzw. Faserstäbe oder gepressten Faserstäbe einkoppelbar sind, ohne dass erhöhte Einkoppelverluste entstehen. Andererseits kann am distalen eine weitwinklige Ausleuchtung ohne zusätzlich erforderliche Optik erreicht werden, was insbesondere endoskopische Untersuchungen bevorzugt. Eine optimale Ausleuchtung bei den derzeit üblichen Kamera-Sichtwinkeln (üblicherweise 120° diagonal) kann dann erzielt werden, wenn die Glasfasern, Faserstäbe oder gepressten Faserstäbe einen Akzeptanzwinkel 2α von mindestens 120° bzw. eine NA von mindestens 0,86 aufweisen..

Eine Ausführungsform des Diagnose-, Operations- und/oder Therapiegeräts und/oder des Lichtleiters, bei welchem das Faserbündel mindestens eine Glasfaser, insbesondere eine Glasfaser umfassend ein mehrkomponentiges silikatisches Glas oder eine Glasfaser aus einem mehrkomponentigen silikatischen Glas, umfasst oder bevorzugt als Glasfaserbündel, insbesondere als Glasfaserbündel umfassend Glasfasern umfassend ein mehrkomponentiges, silikatisches Glas oder aus einem mehrkomponentigen silikatischen Glas oder aus Glasfasern aus einem mehrkomponentigen silikatischen Glas, ausgebildet ist, ist dabei besonders vorteilhaft. Denn mit solchen Glasfasern sind die optischen Eigenschaften des Glasfaserbündels und mithin des Lichtleiters bzw. des Diagnose-, Operations- und/oder Therapiegeräts besonders flexibel einstellbar. Weiterhin weisen solche Lichtleiter, die auf Glasfasern, basieren, eine deutlich höhere Temperaturbeständigkeit als eine polymere optische Faser (POF) auf. Dies ist insbesondere dann relevant, wenn eine besonders gute Einkoppeleffizienz realisiert werden soll, beispielsweise dann ein dünnes Faserbündel aus oder umfassend Glasfasern direkt auf einen LED-Chip kontaktiert werden oder sehr nahe an einen solchen Chip herangeführt werden. Eine polymere optische Faser bzw. ein Faserbündel aus oder umfassend polymere optische Fasern würde einer solchen thermischen Belastung jedoch nicht standhalten, sondern es würden zum Schmelzen der Fasern kommen.

Gemäß einer weiteren bevorzugten Ausführungsform des Diagnose-, Operations- und/oder Therapiegeräts bzw. des Lichtleiters umfasst der Lichtleiter ein Faserbündel umfassend eine Glasfaser, welche eine numerische Apertur gegenüber Luft von größer als 0,80, bevorzugt größer als 0,85 aufweist, und wobei vorzugsweise der Kern der Glasfaser ein glasiges Material umfasst, dessen Zusammensetzung ausgewählt ist aus den vorstehend aufgeführten Glaszusammensetzungen und Glaszusammensetzungsbereichen für Kerngläser. Insbesondere kann der Kern der Glasfaser überwiegend, also zu mindestens 50 Gew.-%, oder im Wesentlichen, also zu mindestens 90 Gew.-%, oder sogar vollständig aus einem solchen glasigen Material bestehen. Dies ist vorteilhaft, weil auf diese Weise eine sehr gute Ausleuchtung des Kamera-Gesichtsfeldes (hier insbesondere für sogenannte C-MOS-Kameras mit z.B. 1 x 1 mm2 Fläche) realisiert werden kann.

Gemäß einer weiteren Ausführungsform des Diagnose-, Operations- und/oder Therapiegeräts bzw. des Lichtleiters umfasst das Faserbündel mindestens eine Glasfaser, wobei das Kern- und/oder das Mantelglas der Glasfaser bis auf unvermeidliche Spuren frei ist von Blei und/oder Antimon und/oder Arsen und/oder von sonstigen Schwermetallen.

Gemäß einer nochmals weiteren Ausführungsform des Diagnose-, Operations- und/oder Therapiegeräts bzw. des Lichtleiters weist mindestens eine Faser des Faserbündels, bevorzugt eine Faser umfassend ein Glas oder aus einem Glas, besonders bevorzugt umfassend ein mehrkomponentiges silikatisches Glas oder aus einem mehrkomponentigen silikatischen Glas, eine Polymerschicht als äußere Hülle aufweist, welche bevorzugt aus einem thermoplastischen Material gebildet ist und einen Schmelzbereich im Temperaturbereich von mind. 80°C bis maximal 400°C, bevorzugt 80°C bis maximal 250°C, besonders bevorzugt mindestens 120°C bis 200°C, aufweist. Insbesondere können für fluorierte oder Fluor-Polymere Verarbeitungstemperaturen von 80°C bis 400°C möglich sein. Eine solche Ausgestaltung ist besonders vorteilhaft bei Ausführungen des Lichtleiters bzw. des Diagnose-, Operations- und/oder Therapiegeräts, die im Hinblick auf die Einkoppeleffizienz optimiert sind und bei denen das proximale Ende des Lichtleiters sehr nahe an eine Lichtquelle herangeführt wird oder diese sogar kontaktiert. Eine solche Lichtquelle kann beispielsweise ein LED-Chip sein oder einen solchen LED-Chip umfassen.

Gemäß einer weiteren Ausführungsform des Lichtleiters bzw. des Diagnose-, Operations- und/oder Therapiegeräts ist die Terminierung des Faserbündels an dem wenigstens einen, bevorzugt am distalen Endes des Faserbündels durch Aufbringen und Aushärten eines dünnflüssigen transparenten Klebers und/oder Gießharzes ausgebildet, wobei der Kleber und/oder das Gießharz mittels UV-Licht und/ oder thermisch aushärtbar ist, und/oder ist die Terminierung des Faserbündels an dem wenigstens einen, bevorzugt am distalen Ende in der Form ausgeführt, dass sie erhalten ist durch einen Heißprozess, bevorzugt durch einen Heißpress-Prozess.

Gemäß einer weiteren Ausführungsform des Diagnose-, Operations- und/oder Therapiegeräts bzw. des Lichtleiters weist der Lichtleiter wenigstens eines der folgenden Merkmale auf:
- Die mindestens eine Endfläche liegt als geschliffene und/oder als polierte und/oder als geschnittene, vorzugsweise als lasergeschnittene, Endfläche vor,
- die zumindest eine terminierte Endfläche weist eine Oberflächenrauigkeit Ra von ≤ 1,0 µm, vorzugsweise ≤ 0,5 µm, besonders bevorzugt ≤ 0,1 µm auf,
- die wenigstens eine terminierte Endfläche umfasst einen transparenten Kunststoff, welcher einen Brechungsindex aufweist, der im Wesentlichen dem des Kernmaterials der Fasern des Faserbündels entspricht,
- die zumindest eine terminierte Endfläche weist einen kreisrunden, nierenförmigen, halbmondförmigen oder sonstigen unregelmäßig geformten Querschnitt auf,
- das Mantelmaterial des Lichtleiters weist an seiner Oberfläche zumindest abschnittsweise eine niedrige Oberflächenenergie, wobei eine niedrige Oberflächenenergie eine Oberflächenenergie von weniger als 40 mN/m, bevorzugt von weniger als 30 mN/M und besonders bevorzugt von weniger als 20 mN/m, ist, auf
- der Mantel des Lichtleiters liegt zumindest abschnittsweise beschichtet und/oder vorbehandelt vor, sodass vorzugsweise mittels physikalischer und/ oder chemischer Vorbehandlung zumindest abschnittsweise die Gleiteigenschaften der Oberfläche des Mantelmaterials verbessert sind.

Es ist vorteilhaft, wenn die mindestens eine terminierte Endfläche als geschliffene und/oder polierte und/oder geschnittene, vorzugsweise als lasergeschnittene, Endfläche vorliegt und/oder als Endfläche mit einer Oberflächenrauigkeit von Ra von ≤ 1,0 µm, vorzugsweise ≤ 0,5 µm, besonders bevorzugt ≤ 0,1 µm.

Weiterhin kann es bevorzugt sein, dass der vom Mantel umfasste Kunststoff, vorzugsweise der transparente Kunststoff, der mindestens einen terminierten Endfläche, vorzugsweise sowohl der terminierten proximalen und/ oder der terminierten distalen Endflächen, eine typische Oberflächenrauigkeit Ra von ≤ 1,0 µm, vorzugsweise ≤ 0,5 µm, besonders bevorzugt ≤ 0,1 µm aufweist. Denn mit solchen Ausführungsformen des Lichtleiters lassen sich Streuverluste an der Oberfläche, also der Endfläche, minimieren, welche ansonsten zu einer Reduzierung der Beleuchtungsstärke bei Lichtleitern führen würden. Bei Bildleitern kann damit eine scharfe Abbildung des beleuchteten Objekts erreicht werden. Insbesondere können solche geringen Rauigkeiten der Oberfläche für polierte Endflächen erzielt werden. Es kann aber auch ausreichend sein, wenn eine größere Rauigkeit erzielt wird - dies gilt insbesondere dann, wenn eine eher diffuse Lichtverteilung gewünscht ist.

Gemäß einer weiteren Ausführungsform des Lichtleiters bzw. des Diagnose-, Operations- und/oder Therapiegeräts nach der vorliegenden Offenbarung ist die wenigstens eine terminierte Endfläche so ausgestaltet, dass sie einen transparenten und/oder auch transluzenten und/oder opaken und/oder eingefärbten Kunststoff umfasst., welcher einen Brechungsindex aufweist, der im Wesentlichen dem des Kernmaterials der Fasern des Faserbündels entspricht. Dies bedeutet, dass die Terminierung mindestens einer Endfläche des Lichtleiters also so ausgebildet sein kann, dass sie zumindest teilweise Mantelmaterial umfasst. Dies bedeutet mit anderen Worten, dass das Mantelmaterial also ein Teil der terminierten Endfläche des Lichtleiters ist oder zumindest sein kann, wobei in diesem Fall der vom Mantel umfasste Kunststoff und/oder der Mantel selbst opak ausgebildet sein kann. Dies bedeutet mit anderen Worten, dass der Kunststoff, der die Fasern umgibt oder infiltriert, opak sein kann, sowie auch der Mantel oder Schlauch, der dieses zumindest teilweise an oder nahe den Endflächen oder wenigstens einer Endfläche infiltrierte Faserbündel umgibt, auch opak sein kann.

Vorteilhaft ist eine solche Ausgestaltung deshalb, weil auf diese Weise Streuverluste sowie der Einfluss von Streulicht auf die Kamera minimiert werden können, was bei den Lichtleitern zu einer Steigerung der Beleuchtungsstärke führt und bei Bildleitern Artefakte infolge von Reflektionen unterdrückt.

Unter einem im Wesentlichen gleichen Brechungsindex bzw. einem entsprechenden Brechungsindex wird verstanden, dass die Abweichung zwischen dem Brechungsindex der Fasern oder den Faserkomponenten und dem klar transparenten Kunststoff maximal ± 0,1, besonders bevorzugt maximal ± 0,05 beträgt. Auf diese Weise können bereits gute Ergebnisse erzielt werden; beispielsweise sind bei einer maximalen Abweichung von ± 0,05 die Brechungsindices bereits nahezu perfekt angepasst, so dass die Reflexionsverluste bei den Lichtleitern vernachlässigt werden können. Bei den Bildleitern können insbesondere Geisterbilder infolge von Mehrfachreflexionen ausgeschlossen werden.

Der Querschnitt der Terminierung kann unterschiedliche Formen aufweisen. In der Regel wird die Terminierung dabei in der Form erfolgen, dass die terminierte Fläche des Lichtleiters im Rahmen der Messgenauigkeit als kreisförmig zu bezeichnen ist, mithin rund.

Besonders bevorzugt ist eine Ausführungsform des Diagnose-, Operations- und/oder Therapiegeräts bzw. des Lichtleiters in der Form, dass der Querschnitt der Terminierung anstelle eines kreisrunden Querschnitts einen nierenförmigen, halbmondförmigen oder sonstigen unregelmäßig geformten Querschnitt aufweist. Insbesondere zur mechanischen Anbindung an andere Komponenten des Endoskops kann es nämlich weiterhin vorgesehen sein, dass die proximale und/ oder die distale Endfläche zudem jeweils eine mechanische Schnittstelle in Form einer Kontur der terminierten Endfläche aufweist, welche aus Kunststoff besteht oder mittels Kunststoff-Spritzguß an den Lichtleiter angeformt ist, wobei dieser Kunststoff sich vom transparenten Kunststoff der proximalen und/oder distalen Endfläche bzw. vom Kunststoff, welcher vom Mantel umfasst wird, zumindest teil- oder abschnittsweise hinsichtlich Material, Transparenz und/ oder Farbe unterscheiden kann. So lassen sich beispielsweise Kragen oder Absätze, aber auch Hinterschnittbereiche erzeugen, mit denen sich der Lichtleiter mit einem Handstück und/ oder eines Schaftes des Endoskops verbinden lässt. Unter anderem können damit auch Rastverbindungen realisiert werden, die eine schnelle Montage ermöglichen, was wiederum die Herstellkosten senken kann.

Liegt der Bildleiter so vor, dass das Mantelmaterial des Lichtleiters an seiner Oberfläche zumindest abschnittsweise eine niedrige Oberflächenenergie aufweist und/oder so, dass der Mantel des Lichtleiters zumindest abschnittsweise beschichtet und/oder vorbehandelt vorliegt, sodass vorzugsweise mittels physikalisch und/ oder chemischer Vorbehandlung zumindest abschnittsweise die Gleiteigenschaften der Oberfläche des Mantelmaterials verbessert sind, ist dies vorteilhaft, denn auf diese Weise kann der sehr dünne Lichtleiter nach Ausführungsformen leichter in ein Diagnose-, Operations- und/oder Therapiegerät nach Ausführungsformen eingeführt werden. Insbesondere ist dann für das Einführen eine geringe Kraft erforderlich. Insbesondere kann es vorteilhaft sein, wenn die vorteilhaften Oberflächeneigenschaften des Mantels, also die Beschichtung und/oder die Vorbehandlung zur Erzielung einer niedrigen Oberflächenenergie nicht nur abschnittsweise auf der Oberfläche des Mantels vorliegen, sondern auf dessen gesamter Oberfläche.

Eine Möglichkeit zur zumindest abschnittsweise erfolgenden Beschichtung und/oder Vorbehandlung stellen beispielsweise chemische und/oder physikalische Beschichtungs- und/ oder Aktivierungsverfahren vor, mittels denen beispielsweise auch sehr niedrige Oberflächenenergien von weniger als 40 mN/m, bevorzugt von weniger als 30 mN/m und besonders bevorzugt von weniger als 20 mN/m erzielt werden können. Beispielsweise ist es, insbesondere wenn der Mantel aus Silikon ausgebildet oder wenn der Mantel ein silikonhaltiges Material umfasst, möglich, eine Fluorbegasung des Mantels durchzuführen. Es ist aber auch möglich, eine Beschichtung umfassend Parylene aufzubringen. Insbesondere für Silikone kann auch eine Beschichtung mit TopCoat, einem Produkt der Nedform BV/ NL, erfolgen.

Gemäß einer weiteren Ausführungsform des Diagnose-, Operations- und/oder Therapiegeräts und/oder des Lichtleiters umfasst der Mantel neben einem ersten Kunststoffmaterial ein weiteres Kunststoffmaterial und/oder die terminierte Endfläche umfasst ein erstes Kunststoffmaterial und der Mantel umfasst ein weiteres Kunststoffmaterial. Mit anderen Worten kann die Zusammensetzung der terminierten Endfläche und des Mantels hinsichtlich des Kunststoffmaterials unterschiedlich sein. Dies kann insbesondere dann vorteilhaft sein, wenn der Mantel als extrudierter Mantel ausgeführt ist, denn dies ist eine besonders kostengünstige Ausführung eines Mantels und erlaubt hinsichtlich der Gestaltung des Lichtleiters und entsprechend auch des Diagnose-, Operations- und/oder Therapiegeräts viele Freiheitsgrade. Gemäß einer Ausführungsform umfasst der Mantel ein weiteres Kunststoffmaterial und ist als extrudierter Mantel ausgeführt.

Bevorzugt kann gemäß einer Ausführungsform der vom extrudierten Mantel umfasste Kunststoff einen zumindest teil- oder abschnittsweise transparenten, transluzenten, also durchscheinenden, opaken, also undurchsichtigen, und/oder eingefärbten, mithin absorbierenden, Kunststoff umfassen. Mit anderen Worten ist es gemäß dieser Ausführungsform auch möglich, dass nicht der gesamte Mantel transparent ausgebildet ist, was vorteilhaft sein kann, da auf diese Weise die Leuchteigenschaften des Lichtleiters gezielt einstellbar sind. Damit kann nämlich beispielsweise mit einer seitlich abstrahlenden Lichtleitfaser eine seitliche Beleuchtung an einem Diagnose-, Operations- und/oder Therapiegerät, wie einem Endoskop, ermöglicht werden.

Gemäß einer weiteren Ausführungsform des Diagnose-, Operations- und/oder Therapiegeräts besteht der Lichtleiter aus flexiblen oder semi-flexiblen Faserbündeln und der Mantel ist zumindest teil- oder abschnittsweise als starre Hülle ausgebildet. In diesem Fall kann damit vorteilhaft beispielsweise ein Schaft für ein starres Endoskop realisiert werden. Damit lassen sich besonders kostengünstig flexible oder starre Einweg-Endoskope realisieren.

Gemäß einer weiteren Ausführungsform des Diagnose-, Operations- und/oder Therapiegeräts und/oder des Lichtleiters besteht der Lichtleiter ausgezogenen und/oder gepressten Faserstäben und bildet einen starren Lichtleiter aus. Auch damit lassen sich besonders kostengünstig gerade starre Einweg-Endoskope realisieren.

Gemäß einer weiteren Ausführungsform des Diagnose-, Operations- und/oder Therapiegeräts und/oder des Lichtleiters ist ein zuvor extrudierter Mantel (in bestimmten Abständen oder ein entsprechender Faserbündelabschnitt, welcher mit einem als Schlauch oder Schrumpfschlauch ausgebildetem Mantel umgeben ist, entsprechend der finalen Bauteillänge geteilt und der Mantel oder der Schlauch oder Schrumpfschlauch ist gegenüber dem Faserbündel gelängt und die dabei entstehende Kavität mit optisch transparenten, beispielsweise einem klar transparenten und vorzugsweise selbstnivellierendem, Kunststoff aufgefüllt oder ein vorgefertigtes klar transparentes Kunststoffteil oder ein Lichtleitstab oder Faserstab aus Glas oder Kunststoff ist in die Kavität eingesetzt und fixiert. Damit lassen sich entsprechende Lichteintrittsbeziehungsweise Lichtaustrittsflächen realisieren, die beispielsweise auch eine hinreichend glatte Oberfläche ausbilden können.

Gemäß einer nochmals weiteren Ausführungsform des Diagnose-, Operations- und/oder Therapiegeräts und/oder des Lichtleiters ist dabei bevorzugt der Mantelabschnitt, Schlauch- oder Schrumpfschlauchabschnitt, der die Kavität ausbildet, verformt und bildet eine bestimmte Lichteintritts- oder Lichtaustrittskontur nach Aushärten des Kunststoffes oder nach Einsetzen des Kunststoffteils oder des Lichtleitstabs aus.

Vorteilhaft weisen gemäß einer weiteren Ausführungsform des Diagnose-, Operations- und/oder Therapiegeräts bzw. des Lichtleiters die proximalen oder distalen Endflächen des Lichtleiters weitere aktive elektronische Elemente in Form von LEDs, Laser-Dioden, Sensoren oder Kamera-Chips auf, welche an die terminierte Endfläche verbindbar und/oder mittels einer Rastverbindung ansteckbar sind. Auf diese Weise wird vorteilhaft eine gute Verbindung zwischen dem Lichtleiter und weiteren Komponenten des Diagnose-, Operations- und/oder Therapiegeräts erzielt.

Gemäß einer nochmals weiteren Ausführungsform des Diagnose-, Operations- und/oder Therapiegeräts und/oder des Lichtleiters sind die proximalen und/ oder distalen Endflächen des Lichtleiters als optisches Element zur Erzielung einer bestimmten Strahlformung ausgeführt und weisen dabei eine plane, konvexe, konkave Fläche oder eine hinsichtlich ihrer Topographie beliebig gestaltete Freiformfläche auf.

Besonders vorteilhaft ist es dabei, wenn die Bündel oder Einzelfasern zumindest teil- oder abschnittsweise mit einem Mantel, Schlauch, Schrumpfschlauch oder Netzschlauchgewebe umgeben sind oder mittels eines Schaftes des Endoskops geschützt ist. Dies erhöht die mechanische Robustheit des Systems.

Dabei kann vorgesehen sein, dass der Mantel aus einem weiteren Kunststoffmaterial besteht und als extrudiertes Kabel ausgeführt ist. Derartige Kabel lassen sich besonders kostengünstig in einem Endlos-Prozess herstellen.

Insbesondere können für das Mantelmaterial in den oben genannten Ausführungsvarianten kostengünstige, weniger temperaturstabile Kunststoffe eingesetzt werden, da bei den Single-Use-Anwendungen keinerlei insbesondere thermische/ chemische Aufbereitungsprozesse, wie Autoklavieren (typ. 130 bis 140°C in gesättigtem Wasserdampf) und/ oder Thermodesinfektor-Prozessen (bis 95°C, Reinigungsmittel mit pH 11) erforderlich sind. I.d.R. werden zur Sterilisation von Einweg-Instrumenten Ethylenoxid-Begasungen oder zum Teil Plasma-basierte Gas-Sterilisationen (STERAD, mit Wasserstoffperoxid und Plasma, oder STERIS, nur mit Wasserstoffperoxid) eingesetzt, welche bei max. 60° C stattfinden.

Gemäß einer Ausführungsform des Diagnose-, Operations- und/oder Therapiegeräts umfasst dieses elektronische Elemente in Form von LED als Beleuchtungslichtquelle. Diese LED sind als Lichtquelle dem proximalen Ende des Lichtleiters zugeordnet und sind das proximalen Endes des Lichtleiters und die Lichtquelle zueinander mit einem nur geringen Abstand voneinander positioniert. Dies ist vorteilhaft, denn so ist eine besonders hohe Einkoppel-Effizienz ermöglicht, was sich besonders vorteilhaft hinsichtlich der Beleuchtungsstärke am distalen Ende des Lichtleiters bemerkbar macht. Als LEDs können neben Weißlicht-LEDs auch RGBW-LEDs zum Einsatz kommen, bei denen zwischen verschiedenen Farben umgeschaltete werden kann. Dies ermöglicht neben einer normalen Betrachtung von Gewebe auch bestimmte diagnostische Untersuchungen, bei denen das Gewebe mit bestimmten Wellenlängen untersucht wird. Denkbar ist auch die Kombination von Weißlicht- beziehungsweise RGBW-LEDs mit LEDs, die im tief blauen Spektralbereich (zum Beispiel 405 nm) oder im nahen UV-Bereich emittieren. Damit können auch Fluoreszenz-Anregungen ermöglicht werden. Hinsichtlich eines Wärmemanagements kann vorgesehen sein, dass die LEDs mittels Metallstifte mit Wärmesenken im Handstück des Endoskops thermisch verbunden sind.

Vorteilhaft kann es sein, wenn die proximalen und/ oder distalen Endflächen als optisches Element zur Erzielung einer bestimmten Strahlformung ausgeführt sind, und dabei eine plane, konvexe, konkave Fläche oder eine hinsichtlich ihrer Topographie beliebig gestaltete Freiformfläche aufweisen. Durch eine entsprechende Werkzeuggestaltung kann beispielsweise zur besseren Lichteinkopplung die Terminierung der proximalen Endfläche mit Kondensor-Linsen ausgestattet sein, um beispielsweise das Licht der in der Regel eher breit abstrahlenden LEDs zu bündeln und entsprechend der numerischen Apertur der Fasern (zwischen 0,55 und 0,70; zum Beispiel SCHOTT PURAVIS® GOF70 mit einer numerischen Apertur von 0,57, SCHOTT PURAVIS® GOF85 mit einer numerischen Apertur von 0,68) in diese einzukoppeln. Eine entsprechende Ausbildung einer konvexen Linse am distalen Ende kann auch vorteilhaft genutzt werden, beispielsweise eine abbildende Optik für den Kamera-Chip zu realisieren. Weiterhin kann auch eine Weitwinkel-Abstrahlcharakteristik, zum Beispiel mit sphärischer oder ringförmiger Abstrahlcharakteristik, am distalen Ende des Lichtleiters mit derart ausgebildeten optischen Elementen ermöglicht werden. Mit einer sphärischen Abstrahlcharakteristik kann beispielsweise eine homogene Ausleuchtung von Körperhohlräumen erfolgen.

All die zuvor ausgeführten Beispiele sind geeignet, entsprechend kostengünstige faseroptische Bauteile beziehungsweise Baugruppen bereitzustellen, welche in flexiblen oder starren Einweg-Endoskope verbaut werden können. Der Sammelbegriff Einweg-Endoskope umfasst hier alle medizintechnische Geräte mit denen einerseits Licht in das Innere des Körpers geleitet wird und anderseits mittels Optiken, Bildleitern oder Kamera-Chips eine Bildinformation an den Operateur ausgegeben wird. Dies können beispielhaft Angioskope für Gefäßuntersuchungen mit flexiblem Endoskop, Laparoskope für Untersuchungen in der Bauchhöhle und Arthroskope für Gelenkuntersuchungen mit je starrem Endoskop, sowie Ohr-Endoskop, Rhino-Endoskop, Sinuskop oder Osopharyngoskop für HNO-Untersuchungen mit jeweils starrem Endoskop sein.

Hier können die zuvor beschriebenen Ausführungsvarianten der Lichtleiter in einem Handstück des Endoskops integriert werden und können, teilweise direkt einen flexiblen Abschnitt oder einen Schaft des Endoskops ausbilden, je nach Bauart des Endoskops. Durch den Wegfall der teils sehr aufwendigen Schleif- und Polierprozesse und durch die erleichterte Montage selbst können damit Kosten eingespart werden.

Eine weitere Verwendung insbesondere der Lichtleiter, wie sie zuvor in den verschiedenen Ausführungsvarianten beschrieben wurden, sieht neben dem Einsatz im Medizingeräte-Bereich auch den Einsatz für In-vitro-Diagnostik-Geräte vor. Dabei können derartige Lichtleiter auch als Detektorlichtleiter eingesetzt werden. Hier werden beispielsweise oft eine große Zahl derartiger Beleuchtungs- beziehungsweise Detektorlichtleiter in einem Gerät zum Beispiel zur parallelen Untersuchungen an Blutproben verwendet. Hier sind insbesondere die Kostenvorteile, sei es infolge einer Reduzierung des Montageaufwandes oder der Integration von Zusatzfunktionen, zu nennen. Eine biokompatible Ausführung der Kunststoffe kann hier direkt genutzt werden um beispielsweise Blutproben oder Zellkulturen unmittelbar in Kontakt mit den Beleuchtungs- bzw. Detektorlichtleitern zu bringen. Zudem werden mit den zuvor beschriebenen Glas- oder Quarzfasern aufgrund Ihrer Vorteile bei der optischen Übertragung spektroskopische Untersuchungen und/ oder auch Untersuchungen mittels Fluoreszenzanregung ermöglicht.

Derartige Lichtleiter, wie sie zuvor in den unterschiedlichen Ausprägungen beschrieben wurden, lassen sich auch in industriellen und/oder Konsumer-Produkten außerhalb eines medizintechnischen Umfeldes verwenden, bei denen es insbesondere um eine kostengünstige Bereitstellung solcher Lichtleiter geht, die zudem noch optimale optische Eigenschaften aufweisen müssen. Hier sind folgende Verwendungsbeispiele unter anderem zu nennen: Beleuchtungslichtleiter bzw. Lichtleiter in Hausgeräten (Herde, Spülmaschinen, Kühl-/Gefrier-Schränke, Backöfen etc.) beziehungsweise Küchen-Klein-Geräte (Mixer, Toaster, Auftisch-Kochgeräten, Kaffee-Automaten etc.) zum Beispiel zur Kenntlichmachung von Betriebszuständen und/ oder zur Beleuchtung von Garräumen oder Innenräumen, insbesondere dann wenn diese mit Lebensmitteln in Kontakt kommen; Home-Ambiente-Beleuchtung; Automotive Exterieur-/Interieur-Beleuchtung; Maschinenbeleuchtung.

Im Folgenden wird die Erfindung anhand von Beispielen näher erläutert.

### Beispiel 1

Gemäß einer Ausführungsform des Diagnose-, Operations- und/oder Therapiegeräts bzw. des Lichtleiters umfasst der Lichtleiter mindestens zwei optische Fasern, wobei die optischen Fasern ein Faserbündel bilden. Das Faserbündel weist einen Durchmesser zwischen mindestens ca. 0,4 mm bis höchstens ca. 2,0 mm Durchmesser auf. Hierbei ist der äußere Durchmesser des Faserbündels gemeint.

Der Lichtleiter umfasst gemäß dieser Ausführungsform einen Mantel, welcher als sehr dünner Schrumpfschlauch mit einer Wandstärke ausgebildet ist, die im Bereich von typischerweise mindestens ca. 5 µm und höchstens ca. 30 µm liegt. Dieser Mantel bzw. der Schrumpfschlauch kann hier sowohl transparent als auch opak ausgebildet sein. Gemäß der vorliegenden Ausführungsform ist der Mantel hier so ausgebildet, dass er das Faserbündel vollständig über die gesamte Länge, umgibt. Jedoch ist es allgemein, ohne Beschränkung auf das hier konkret dargestellte Beispiel, möglich, dass der Mantel das Faserbündel lediglich abschnittsweise umgibt.

Der Lichtleiter weist hier ein proximales und ein distales Ende auf, wobei allgemein unter einem proximalen Ende das Ende des Lichtleiters verstanden wird, welches einer Lichtquelle zugeordnet ist, beispielsweise mit einer Lichtquelle formschlüssig verbunden ist. Allgemein, ohne Beschränkung auf das hier beschriebene Beispiel einer Ausführungsform des Lichtleiters und/oder des Diagnose-, Operations- und/oder Therapiegeräts weisen sowohl der Lichtleiter als auch das Faserbündel sowohl ein proximales, also eine Lichtquelle oder Beleuchtungslichtquelle, zugeordnetes Ende auf als auch ein distales Ende, nämlich dasjenige Ende des Lichtleiters bzw. des Faserbündels, welches nicht der Lichtquelle, sondern dem zu beleuchtenden Ort zugeordnet ist.

Mindestens eine der Endflächen des Lichtleiters ist hier terminiert, vorzugsweise ist hier das distale Ende terminiert, wobei die Terminierung hier so erfolgt, dass das Faserbündel, vorzugsweise zumindest teilweise innerhalb des Schrumpfschlauchs, mit einem dünnflüssigen transparenten Kleber oder Gießharz verklebt wird.

Beispielsweise ist es möglich, dass der transparente Kleber oder das Gießharz ausgebildet ist als Epoxid-Gießharz oder -Kleber oder als Raumtemperatur-vernetzendes Silikon oder als heißvernetzendes Flüssigsilikon oder als thermisch oder UV-vernetzendes Acrylat-Gießharz oder als Polyurethan-Gießharz oder als Polyester-Gießharz oder aus Mischung und/oder Kombinationen dieser Materialien gebildet ist oder solche Materialien und/oder Mischungen hiervon umfasst.

Insbesondere kann der transparente Kleber als UV-Kleber ausgebildet sein, also als Kleber, der mittels UV-Strahlung aushärtbar ausgebildet ist. Vorteilhaft ist hierbei, dass lange Kleberaushärtezeiten vermieden werden können.

Es ist gemäß der vorliegenden Ausführungsform möglich, dass der Mantel in Form eines Schrumpfschlauchs um das Faserbündel extrudiert wird und dann auf das Faserbündel geschrumpft wird.

### Beispiel 2

Gemäß einer weiteren Ausführungsform des Diagnose-, Operations- und/oder Therapiegeräts bzw. des Lichtleiters ist der Lichtleiter so ausgebildet, dass er mindestens zwei optische Fasern umfasst, wobei die mindestens zwei optischen Fasern ein Faserbünde bilden. Gemäß dieser Ausführungsform ist der Mantel hier in der Form ausgebildet, dass zunächst das Faserbündel mit einem transparenten oder opaken weichem Harz getränkt und/oder benetzt wird. Es ist möglich, dass das Harz das Faserbündel nur oberflächlich in der Form benetzt, sodass beispielsweise nur die Außenseite von solchen Fasern des Faserbündels, welche am Umfang des Faserbündels angeordnet sind, von dem weichen Harz benetzt werden. Es ist aber auch möglich, dass das Harz nicht nur oberflächennah benetzt. Es ist beispielsweise auch möglich, dass es zu weiterreichenden Benetzung des Faserbündels kommt, insbesondere, dass beispielsweise alle Fasern des Faserbündels von dem weichen Harz benetzt sind. Es sind hierbei alle Zwischenstufen möglich, beispielsweise also, dass die Benetzung des Faserbündels an der Außenseite, also am Umfang des Faserbündels, stärker ist und zum Innern des Faserbündels hin abnimmt.

Das Harz weist vorzugsweise eine geringe Shore-Härte auf, insbesondere eine ShoreA-Härte von weniger als 50 oder sogar von weniger als 40.

Das Harz kann beispielsweise mittels Sprühen, Tauchen oder Gießen aufgebracht werden. Eine dünne Extrusion des Materials um das Faserbündel herum, ohne dass dabei ein Luftspalt ausgebildet wird, ist gleichfalls möglich.

Besonders bevorzugt ist es allgemein, ohne Beschränkung auf das hier konkret beschriebene Beispiel, wenn das Harz ein transparentes Harz ist und/oder wenn das Harz im festen Zustand einen Brechungsindex aufweist, welcher vorzugsweise kleiner als der Brechungsindex des sogenannten Claddings ist.

Beispielsweise ist es gemäß dieses Beispiels möglich, dass das Faserbündel einen Durchmesser von 0,4 mm aufweist und ein Mantel mit Dicke von 25 µm verwendet wird, oder einen Durchmesser von 1 mm mit einem Mantel einer Dicke von 50 µm oder ein Faserbündel mit einem Durchmesser von 2 mm und einem Mantel einer Dicke von 250 µm, oder auch ein Faserbündel mit einer Dicke von 2 mm mit einem Mantel einer Dicke von 25 µm und ein Faserbündel mit einer Dicke von 0,4 mm mit einem Mantel mit einer Dicke von 250 µm versehen wird.

Es könnte gemäß einer nochmals weiteren Ausführungsform auch ein noch dickerer Mantel mit einer Dicke von 500 µm verwendet werden.

Es ergibt sich damit nach einer Ausführungsform also ein Lichtleiter, bei welchem ein Flächenverhältnis der Querschnittsfläche des Lichtleiters zur Querschnittsfläche des Faserbündels zwischen 1,025 und 2,64 vorliegt. Allgemein kann das oben genannte Flächenverhältnis gemäß einer Ausführungsform in einem Bereich von 1,01 bis 3,0 liegen. Das Flächenverhältnis kann gemäß noch einer Ausführungsform auch unabhängig von der Dicke des Mantels den Lichtleiter charakterisieren. Dazu ist allgemein ein Lichtleiter, umfassend mindestens zwei optische Fasern vorgesehen, wobei die mindestens zwei optischen Fasern ein Faserbündel bilden, und einen die Fasern und/oder das Faserbündel zumindest teil- oder abschnittsweise umgebenden Mantel, wobei das Flächenverhältnis der Querschnittsfläche des Lichtleiters zur Querschnittsfläche des Faserbündels in einem Bereich von 1,01 bis 3,0, vorzugsweise in einem Bereich von 1,025 und 2,64 liegt, wobei der Lichtleiter ein proximales und ein distales Ende und zumindest eine terminierte Endfläche an mindestens einem Ende aufweist, wobei die terminierte Endfläche eine maximale laterale äußere Abmessung aufweist, beispielsweise einen Außendurchmesser, welche höchstens so groß ist wie die maximale äußere laterale Abmessung, beispielsweise der Außendurchmesser, der Querschnittsfläche des Lichtleiters.

### Beispiel 3

Gemäß einer nochmals weiteren Ausführungsform ist es möglich, dass ein Lichtleiter, welcher einen Mantel aufweist, welcher wie in Beispiel 2 ausbildet wird, mit einer Endflächenterminierung entsprechend des Beispiels 1 kombiniert wird, also mittels eines Klebers eine Endflächenterminierung, vorzugsweise am distalen Ende des Lichtleiters, erhalten wird.

Bevorzugt kann bei dieser Ausführungsform ein UV-aushärtendes Kleber und/oder ein UV-aushärtendes Gießharz verwendet werden.

Beispielsweise ist es hierbei möglich, dass ein Faserbündel gemäß einer Ausführungsform nach Beispiel 2, endflächennah in eine Form gepresst wird, was beispielsweise mittels Pressen in oder zwischen entsprechenden Formen erfolgen kann. Dann kann ein UV-aushärtendes Kleber und/oder ein UV-aushärtendes Gießharz von oben kommend auf die Endfläche vergossen werden. Durch eine gezielte UV-Beaufschlagung ist es möglich, die Eindringtiefe des Klebers bzw. Gießharzes und somit die Länge der Versteifung des Faserbündels gezielt zu definieren. Insbesondere kann die Eindringtiefe mehrere Millimeter betragen, insbesondere bis zu maximal 10 mm.

Eine solche Ausführungsform ist insbesondere auch deshalb vorteilhaft, weil auf diese Weise auch unrunde Endflächengeometrien erzeugt werden können, beispielsweise halbmondförmige oder nierenförmige Geometrien.

Allgemein ist es jedoch auch möglich, dass ein Kleber und/oder ein Gießharz verwendet wird, welches thermisch aushärtet. Auch in diesem Fall kann eine spezielle Endflächengeometrie erzeugt werden und es ist weiterhin auch möglich, die Eindringtiefe und damit die Länge der Versteifung des Lichtleiters mit solchen Materialien einzustellen.

Es ist allgemein, ohne Beschränkung auf das hier beschriebene Ausführungsbeispiel, möglich und kann sogar bevorzugt sein, dass der Kleber bzw. das Gießharz so ausgewählt ist, dass ein gewünschter Brechungsindex mit diesem Kleber bzw. diesem Gießharz möglich ist. Damit können also die Brechungsindices von Endflächenterminierung und/oder Mantel und/oder den optischen Fasern aufeinander abgestimmt sein (sogenanntes Matching der Brechungsindices).

Es ist allgemein, ohne Beschränkung auf die hier beschriebene Ausführungsform, möglich und kann sogar bevorzugt sein, dass der Kleber und/oder das Gießharz in der Form als Endflächenterminierung ausgestaltet ist, dass der ausgehärtete Kleber und /oder das Gießharz in Form eines optischen Elements, wie beispielsweise einer Linse, ausgebildet sind.

Vorteilhaft ist eine Ausführung nach einem der beschriebenen Beispiele 2 oder 3 insbesondere auch, weil bei der Verwendung eines thermoplastischen Klebers und/oder Gießharzes eine direkte Heißverformung möglich ist.

Auch können allgemein bei einer solchen Endflächenterminierung Enden erhalten werden, welche gegebenenfalls nachbearbeitbar sind.

### Beispiel 4

Gemäß einer weiteren Ausführungsform des Diagnose-, Operations- und/oder Therapiegeräts bzw. des Lichtleiters umfasst der Lichtleiter optische Fasern umfassend ein optisches Glas, also allgemein Glasfasern, wobei diese Glasfasern ein Kernglas und ein Mantelglas umfassen und an ihrer Außenseite zumindest teilweise mit einer thermoplastischen Polymerschicht versehen sind, wobei das Polymermaterial beispielsweise durch Tauchen, Sprühen oder Extrudieren ohne Luftspalt auf die Faser aufgebracht werden kann.

Ein solches Faserbündel kann insbesondere vorteilhaft mit einer Endflächenterminierung, wie sie beispielhaft in den Beispielen 2 und 3 der vorliegenden Offenbarung beschrieben ist, kombiniert werden. Insbesondere können die Bearbeitungstemperaturen der Kunststoffe der Polymerschicht sowie die Kunststoffe, welche für die Endflächenterminierung verwendet werden, aufeinander abgestimmt ausgewählt werden, sodass beispielsweise die Endflächenterminierung mit dem Faserbündel materialschlüssig verbunden werden kann und/oder dass das Faserbündel und entsprechend der resultierende Lichtleiter in eine spezielle, insbesondere eine unrunde, Form gebracht werden können.

Allgemein ist es auch möglich, für eine solche Ausführungsform auch Fasern zu wählen, welche nur ein Kernglas umfassen. In diesem Fall kann das Cladding, also das Fasermantelmaterial, als Polymer ausgeführt sein. Das Polymercladding weist dann entsprechende optische Eigenschaften auf, insbesondere hinsichtlich des Brechungsindex, wie dies bei üblichen Kern-Fasermantel-Ausgestaltungen von Glasfasern der Fall ist. Insbesondere ist also in diesem Fall der Brechungsindex des Polymers geringer als der des Kernglases.

### Beispiel 5

Es ist weiterhin auch möglich, gemäß einer weiteren Ausführungsform des Diagnose-, Operations- und/oder Therapiegeräts bzw. des Lichtleiters das Faserbündel mit einem dünnen Folienband bzw. einer dünnen Folie zu umwickeln bzw. zu bandieren. Beispielsweise ist ein entsprechender Ansatz beschrieben in der Patentschrift DE 1 596 485 B1. Ein Umwickeln ist auch beschrieben in der DE 10 2006 040 214 B4, allerdings für Hohlfasern für Trennverfahren.

Vorzugsweise umfasst die Folie bzw. das Folienband Polyimid oder PET. Weiterhin sollte das Folienband bzw. die Folie weniger als 30 µm dick, vorzugsweise sogar weniger als 10 µm dick ausgestaltet sein.

Die Folie bzw. das Folienband kann zum erhöhten Schutz vor Schmutz und Feuchtigkeit der darunterliegenden Faser bzw. Faserbündel auch überlappend vorzugsweise 50% der Folienbreite aufgebracht sein. Hierbei kann das Folienmaterial so gestaltet sein, dass durch die Materialeigenschaften eine Selbstanhaftung der überlappenden Folienlagen erfolgt (siehe auch Frischhaltefolie). Alternativ können auch Folienmaterialien verwendet werden die selbstverschweißend oder durch zusätzliche thermische- oder lichttechnische Prozessschritte wie z. B. Sintern bei Folienmaterialien wie PTFE einen Verbund miteinander eingehen.

Die Verfahren Umflechten und Umgarnen können mit einem oder mehreren Filamentsträngen, erfolgen, wobei diese Stränge wiederum aus mehreren unterschiedlichen Materialien bestehen können. Typische Materialien sind Fäden aus Fiberglas, Glasseide, Aramid, beispielsweise erhältlich unter dem Handelsnamen Kevlar ®, Carbon-Fasern, Polyester, Polyamid, beispielsweise erhältlich unter dem Handelsnamen Nylon ®, Polyamid, PTFE, beispielsweise erhältlich unter dem Handelsnamen Teflon ®, rostfreier Edelstahl, Gold. Die Fadenstärken liegen hier im Bereich < 50 µm, typischerweise im Bereich von 20 µm bis 30 µm.

Das Umflechten kann als einfach oder Mehrfachumflechtung, z.B. Kreuzflechtung ausgeführt sein. Diese Umflechtung kann lose bis fest um den Kern gelegt werden.

Auch hier kann dann eine entsprechende Terminierung der Endfläche wie in den Beispiele 2 und 3 beschrieben ausgeführt werden.

Es ist aber auch möglich und kann sogar bevorzugt sein, dass die Endflächenterminierung in der Form erfolgt, dass das Faserbündel und der dieses zumindest abschnittsweise umgebende Mantel mittels Laser-Schneiden terminiert werden. Der Lichtleiter gemäß Ausführungsformen der vorliegenden Offenbarung weist also in diesem Fall eine lasergeschnittene Endfläche auf.

### Beispiel 6

Es ist weiterhin auch möglich, gemäß einer weiteren Ausführungsform des Diagnose-, Operations- und/oder Therapiegeräts bzw. des Lichtleiters, dass das Bündel eine Dicke von 0,5 mm aufweist, wobei der Mantel eine Wandstärke von 0,1 mm aufweist und aus Perfluorethylenpropylen (FEP) ausgebildet ist oder dieses umfasst, sodass ein Lichtleiter mit einer maximalen äußeren lateralen Abmessung von 0,7 mm resultiert. Hierbei ist der Mantel mittels Extrusion hergestellt. Eine solche dünnwandige Extrusion des Mantelmaterials führt zu einem besonders flexiblen Lichtleiter, insbesondere im Vergleich mit einer Ausgestaltung des Mantels in Form einer Folienumwicklung. Insbesondere sind allgemein, ohne Beschränkung auf das hier dargestellte konkrete Beispiel eines Lichtleiters typische Wandstärke bzw. Dicken von extrudierten Mänteln 0,05 mm bis 0,1 mm.

Die Erfindung wird im Folgenden anhand von in den Figuren dargestellten Ausführungsbeispielen näher erläutert. Es zeigen:
Figur 1 schematisch stark vereinfacht ein Einweg-Endoskop, welches als flexibles Endoskop ausgeführt ist,
Figur 2 schematisch stark vereinfacht ein Einweg-Endoskop, welches als starres Endoskop ausgeführt ist,
Figur 3 schematisch den Aufbau eines Lichtleiters,
Figur 4 schematisch den Aufbau des Lichtleiters in einer Schnittansicht und
Figuren 5 und 6 schematisch Schritte eines Heißformgebungsprozesses mit Einzelglasfasern mit einem Polymer-Mantel.

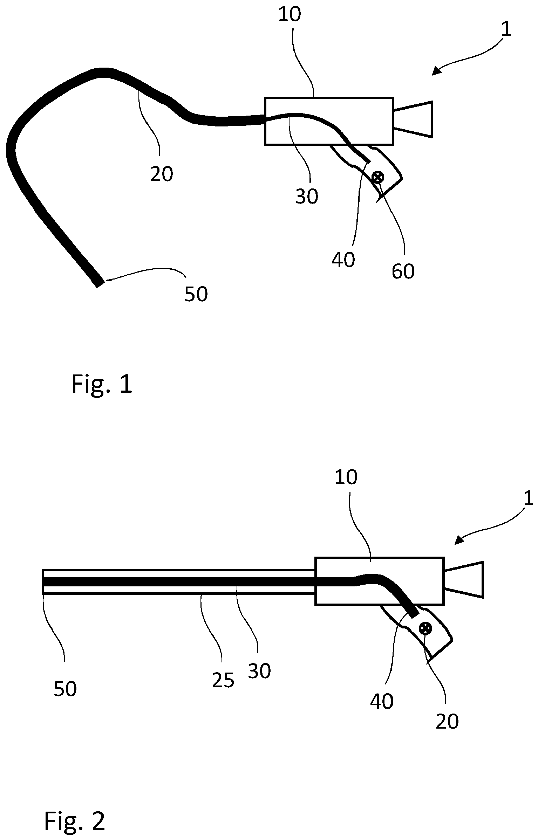

Figur 1 zeigt schematisch den Aufbau eines Endoskops 1 gemäß der vorliegenden Offenbarung. Beispielhaft für ein Diagnose-, Operations- und/oder Therapiegerät ist hier ein einfaches flexibles Endoskop 1 stark vereinfacht dargestellt, welches ein Handstück 10 und einen flexiblen Abschnitt 20 aufweist, wobei der flexible Abschnitt 20 zum Beispiel in einen Körper-Hohlraum einführbar ist. Schematisch dargestellt ist hier ein Lichtleiter 30, der ein proximales Ende 40 bzw. eine proximale Endfläche 30.2 an einer als LED 60 ausgeführten Beleuchtungseinrichtung im Handstück 10 und eine distale Endfläche 30.1 bzw. ein distales Ende 50 am Ende des flexiblen Abschnitts 20 aufweist. Das Licht der LED 60 wird an der proximalen Endfläche 30.2 bzw. am proximalen Ende 40 eingekoppelt und über den Lichtleiter 30 zur distalen Endfläche 30.1 bzw. dem distalen Ende 50 geführt, und kann dann über entsprechende Auskoppeloptiken in das Körperinnere abgestrahlt werden. Nicht dargestellt sind in Figur 1 die bildgebenden Komponenten. Diese können beispielsweise C-MOS-Kameras sein, die an das distale Ende 50 herangeführt sind, und die Bildinformation zu einem ebenfalls nicht dargestellten Monitor elektrisch übertragen. Ebenso denkbar sind faseroptische Bildleiter, die die Bildinformation zu einer Kamera oder direkt zu einer Okularoptik übertragen. Derartige Bildleiter bestehen aus mehreren Tausend feiner, nur wenige µm dicke Einzel-Glasfasern, die entsprechend pixelweise die Bildinformation übertragen.

Je nach Endoskop-Typ und Anwendung sind folgende typischen Abmessungen für derartige Lichtleiter denkbar: Länge zwischen 100 mm und 3000 mm, typ. 500 bis 1000 mm, Lichtleiter-Durchmesser zwischen 0,5 mm und 5 mm, typ. zwischen 1 und 2 mm.

Figur 2 zeigt ebenfalls schematisch und stark vereinfacht ein Endoskop 1, welches als starres Endoskop 1 ausgeführt ist. Der Lichtleiter 30 ist hier in einem starren Schaft 25 geführt. Auch hier sind die bildgebenden bzw. bildübertragenden Komponenten, wie sie zuvor beschrieben wurden, der Übersicht wegen nicht dargestellt.

Im Folgenden werden insbesondere Ausführungsbeispiele beziehungsweise Herstellverfahren beschrieben, die sich auf Lichtleiter 30 beziehen. Grundsätzlich können diese auch auf die Bildleiter übertragen werden.

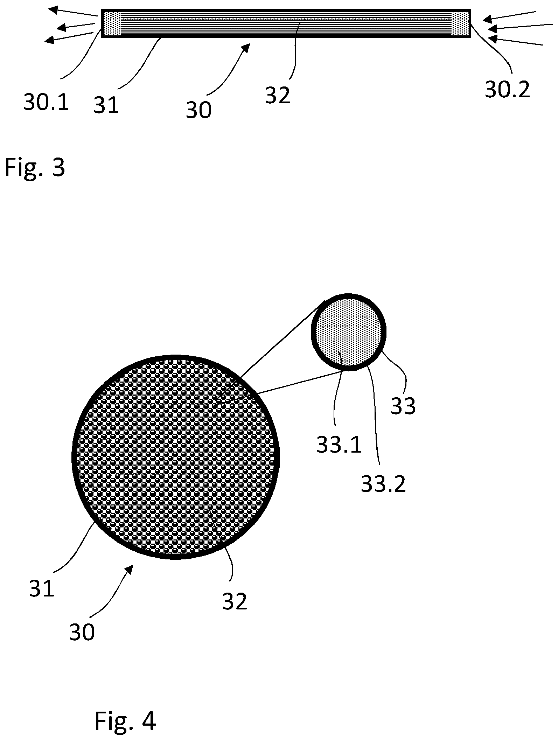

Fig. 3 zeigt schematisch und nicht maßstabsgetreu eine Abbildung eines Lichtleiters 30 nach einer Ausführungsform. Der Lichtleiter 30 umfasst eine distale Endfläche 30.1, welche hier links angeordnet ist, und eine proximale Endfläche 30.2, welche hier rechts angeordnet ist. Weiterhin umfasst der Lichtleiter 30 einen Mantel 31 sowie mindestens zwei Fasern 33 (hier nicht bezeichnet), welche das Faserbündel 32 bilden. Mit anderen Worten ist in Fig. 3 also dargestellt der Lichtleiter 30 umfassend mindestens zwei optische Fasern 33 (hier nicht bezeichnet), wobei die mindestens zwei optischen Fasern 33 ein Faserbündel bilden 32, und einen die Fasern 33 und/oder das Faserbündel 32 zumindest teil- oder abschnittsweise umgebenden Mantel 31, wobei der Lichtleiter 30 eine maximale äußere laterale Abmessung seiner Querschnittsfläche, beispielsweise einen Außendurchmesser, aufweist, welche maximal 500 µm, bevorzugt maximal 200 µm, besonders bevorzugt maximal 100 µm und ganz besonders bevorzugt maximal 50 µm, größer ist als die maximale äußere laterale Abmessung, beispielsweise der Außendurchmesser, der Querschnittsfläche des Faserbündels 32, wobei der Lichtleiter 30 ein proximales und ein distales Ende und zumindest eine terminierte Endfläche 30.2, 30.1 an mindestens einem Ende, vorzugsweise am distalen Ende, aufweist, wobei die terminierte Endfläche 30.2, 30.1 eine maximale laterale äußere Abmessung aufweist, beispielsweise einen Außendurchmesser, welche höchstens so groß ist wie die maximale äußere laterale Abmessung, beispielsweise der Außendurchmesser, der Querschnittsfläche des Lichtleiters 30.

Fig. 4 zeigt eine weitere schematische und nicht maßstabsgetreue Schnittdarstellung eines Lichtleiter 30. Dargestellt ist hier die Querschnittsfläche des Lichtleiters 30, wobei diese Querschnittsfläche hier senkrecht zur Längsachse des Lichtleiters 30 bzw. des Faserbündels 32 bzw. der Längsachse einer Faser 33 liegt. Der Mantel 31 ist hier dünn ausgebildet und umgibt die Fasern des Faserbündels 32 bzw. das Faserbündel 32 zumindest abschnittsweise. Weiterhin ist in Fig. 4 eine schematische Darstellung einer Faser 33 zu sehen, welche vom Faserbündel 32 umfasst ist. Die Faser 33 umfasst einen Faserkern 33.1 und einen Fasermantel 33.2.

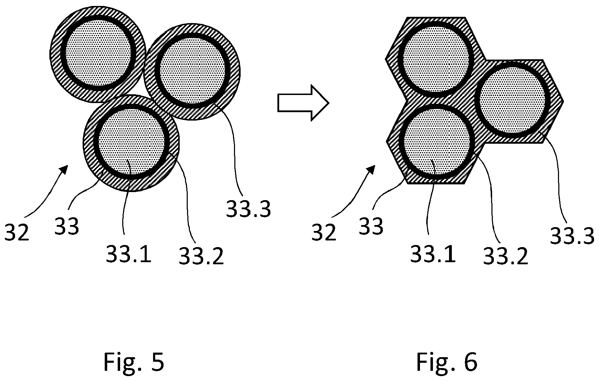

Fig. 5 und 6 zeigen eine schematische Darstellung eines Herstellprozesses eines Faserbündels 32. In Fig. 5 sind drei Fasern 33 zu sehen, welche ein Faserbündel 32 bilden. Die Fasern 33 umfassen jeweils einen Faserkern 33.1 sowie einen Fasermantel 33.2 sowie weiterhin einen Polymermantel 33.3 Dieser Polymermantel wird hier in einem Heißpressprozess so umgeformt, dass er einen Mantel 31 (nicht bezeichnet) bildet, wie dies in der Darstellung in Fig. 6 gezeigt ist. Mit anderen Worten ist in Fig. 6 der Mantel so ausgebildet, dass er die Fasern 33 des Faserbündels umgibt und nicht nur an der Außenseite des Faserbündels 32 angeordnet ist.

### Bezugszeichenliste

- 1: Diagnose-, Operations- und/oder Therapiegerät, hier Endoskop
- 10: Handstück
- 20: flexibler Abschnitt
- 25: Schaft
- 30: Lichtleiter
- 30.1: distale Endfläche
- 30.2: proximale Endfläche
- 31: Mantel
- 32: Faserbündel
- 33: Faser
- 33.1: Faserkern
- 33.2: Fasermantel
- 33.3: Polymer-Mantel
- 40: proximales Ende des Lichtleiters 30
- 50: distales Ende des Lichtleiters 30

## Patentansprüche

1. Lichtleiter (30) umfassend mindestens zwei optische Fasern (33), wobei die mindestens zwei optischen Fasern (33) ein Faserbündel (32) bilden, und
einen die Fasern (33) und/oder das Faserbündel (32) zumindest teil- oder abschnittsweise umgebenden Mantel (31),
wobei der Lichtleiter (30) eine maximale äußere laterale Abmessung seiner Querschnittsfläche, beispielsweise einen Außendurchmesser, aufweist, welche maximal 500 µm, bevorzugt maximal 200 µm, besonders bevorzugt maximal 100 µm und ganz besonders bevorzugt maximal 50 µm, größer ist als die maximale äußere laterale Abmessung, beispielsweise der Außendurchmesser, der Querschnittsfläche des Faserbündels (32),
wobei der Lichtleiter ein proximales und ein distales Ende (40, 50) und
zumindest eine terminierte Endfläche (30.1, 30.2) an mindestens einem Ende (40, 50) aufweist,
wobei die terminierte Endfläche (30.1, 30.2) eine maximale laterale äußere Abmessung aufweist, beispielsweise einen Außendurchmesser, welche höchstens so groß ist wie die maximale äußere laterale Abmessung, beispielsweise der Außendurchmesser, der Querschnittsfläche des Lichtleiters (30).

2. Lichtleiter (30) nach Anspruch 1,
wobei der Mantel (31) des Lichtleiters Kunststoff, bevorzugt einen biokompatiblen und/oder einen sterilisierbaren, vorzugsweise einen mit Ethylenoxid sterilisierbaren, Kunststoff und/oder einen Kunststoff, der nicht toxische Eigenschaften auf menschliche oder tierische Zellkulturen für Einwirkzeiten kleiner einem Tag aufweist, umfasst,
wobei vorzugsweise der Kunststoff ausgewählt ist aus der Gruppe der Cyclo-Olefin-Co-Polymere, Polycarbonate, Polyethylen-Teraphthalaten, Perfluoralkoxy-Polymere, Polyvinylidenfluoride, Polymethylmethacrylate, Polymethylmethacrylimide, Acryl Styrol-Acrylnitril-Co-Polymere oder Raumtemperatur-vernetzende Silikon, heißvernetzende Flüssig-Silikone, UV-vernetzende Silikone, Epoxid-Gießharze oder -Kleber, thermisch oder UV-vernetzende Acrylat-Gießharze, Polyurethan-Gießharze, Polyester-Gießharze oder aus Mischungen und/ oder Kombinationen derselben.

3. Lichtleiter (30) nach einem der Ansprüche 1 oder 2,
wobei der das Faserbündel (32) und/oder die Fasern (33) umgebende Mantel (31) einen Schrumpfschlauch, eine Folie, einen extrudierten Mantel und/oder ein Material mit niederviskosen Fließeigenschaften, welches durch Sprühen, Tauchen, Gießen und/oder Extrusion im Wesentlichen ohne Luftspalt direkt auf das Faserbündel (32) aufbringbar ist, umfasst, wobei jeweils das vom Mantel (31) umfasste Material transparent, transluzent, opak und/oder eingefärbt vorliegen kann, wobei vorzugsweise der das Faserbündel (32) und/oder die Fasern (33) umgebende Mantel (31) mittels Extrusion hergestellt ist und die Wandstärke des Materials maximal 0,15 mm, bevorzugt 0,1 mm und besonders bevorzugt maximal 0,05 mm dick ist, wobei das Mantelmaterial ein Thermoplast, insbesondere ein Fluor-Thermoplast, und/oder ein Hochtemperatur-Kunststoff, bevorzugt ein bei der jeweiligen Verarbeitungstemperatur niedrigviskoser Hochtemperatur-Kunststoff, ist oder umfasst, und/oder ein Compound umfassend einen solchen Thermoplasten oder einen Hochtemperatur-Kunststoff.

4. Lichtleiter (30) nach einem der Ansprüche 1 bis 3,
wobei das Faserbündel (32) eine Glasfaser und/oder eine polymere optische Faser umfasst.

5. Lichtleiter (30) nach einem der Ansprüche 1 bis 4, wobei das Faserbündel (32) mindestens eine Glasfaser, insbesondere Glasfaser umfassend ein mehrkomponentiges silikatisches Glas oder eine Glasfaser aus einem mehrkomponentigen silikatischen Glas umfasst oder bevorzugt als Glasfaserbündel, insbesondere als Glasfaserbündel umfassend Glasfasern umfassend ein mehrkomponentiges, silikatisches Glas oder aus einem mehrkomponentigen silikatischen Glas oder aus Glasfasern aus einem mehrkomponentigen silikatischen Glas, ausgebildet ist.

6. Lichtleiter (30) nach einem der Ansprüche 1 bis 5, wobei der Lichtleiter (30) ein Faserbündel (32) umfassend eine Glasfaser, welche eine numerische Apertur gegenüber Luft von größer als 0,80, bevorzugt von größer als 0,85 aufweist, umfasst.

7. Lichtleiter (30) nach einem der Ansprüche 1 bis 6, wobei das Faserbündel (32) mindestens eine Glasfaser umfasst, wobei das Kern- und/oder das Mantelglas der Glasfaser bis auf unvermeidliche Spuren frei ist von Blei und/oder Antimon und/oder Arsen und/oder von sonstigen Schwermetallen.

8. Lichtleiter (30) nach einem der Ansprüche 1 bis 7,
wobei mindestens eine Faser (33) des Faserbündels (32), bevorzugt eine Faser (33) umfassend ein Glas oder aus einem Glas, besonders bevorzugt umfassend ein mehrkomponentiges silikatisches Glas oder aus einem mehrkomponentigen silikatischen Glas, eine Polymerschicht als äußere Hülle aufweist, welche bevorzugt aus einem thermoplastischen Material gebildet ist und einen Schmelzbereich im Temperaturbereich von mindestens 80°C bis maximal 250°C, bevorzugt mindestens 120°C bis 200°C aufweist.

9. Lichtleiter (30) nach einem der Ansprüche 1 bis 8,
wobei die Terminierung des Faserbündels (32) an dem wenigstens einen, bevorzugt am distalen, Ende (40, 50) des Faserbündels (32) durch Aufbringen und Aushärten eines dünnflüssigen transparenten Klebers und/oder Gießharzes ausgebildet ist,
wobei der Kleber und/oder das Gießharz mittels UV-Licht und/ oder thermisch aushärtbar ist,
und/oder wobei die Terminierung des Faserbündels (32) an dem wenigstens einen Ende des Faserbündels, bevorzugt am distalen Ende, in der Form ausgeführt ist, dass sie erhalten ist durch einen Heißprozess, bevorzugt durch einen Heißpress-Prozess.

10. Lichtleiter (30) nach einem der Ansprüche 1 bis 9, aufweisend wenigstens eines der folgenden Merkmale:
- die mindestens eine Endfläche (30.1, 30.2) liegt als geschliffene und/oder als polierte und/oder als geschnittene, vorzugsweise als lasergeschnittene, Endfläche (30.1, 30.2) vor
- die zumindest eine terminierte Endfläche (30.1, 30.2) weist eine Oberflächenrauigkeit Ra von ≤ 1,0 µm, vorzugsweise ≤ 0,5 µm, besonders bevorzugt ≤ 0,1 µm auf
- die wenigstens eine terminierte Endfläche (30.1, 30.2) umfasst einen transparenten Kunststoff, welcher einen Brechungsindex aufweist, der im Wesentlichen dem des Kernmaterials der Fasern (33) des Faserbündels (32) entspricht
- die zumindest eine terminierte Endfläche (30.1, 30.2) weist einen kreisrunden, nierenförmigen, halbmondförmigen oder sonstigen unregelmäßig geformten Querschnitt auf
- das Mantelmaterial des Lichtleiters (30) weist an seiner Oberfläche zumindest abschnittsweise eine niedrige Oberflächenenergie, wobei eine niedrige Oberflächenenergie eine Oberflächenenergie von weniger als 40 mN/m, bevorzugt von weniger als 30 mN/M und besonders bevorzugt von weniger als 20 mN/m, ist, auf
- der Mantel (31) des Lichtleiters (30) liegt zumindest abschnittsweise beschichtet und/oder vorbehandelt vor, sodass vorzugsweise mittels physikalisch und/ oder chemischer Vorbehandlung zumindest abschnittsweise die Gleiteigenschaften der Oberfläche des Mantelmaterials verbessert sind.

11. Lichtleiter (30) nach einem der Ansprüche 1 bis 10,
wobei der Mantel (31) ein weiteres Kunststoffmaterial umfasst und als extrudierter Mantel ausgeführt ist, wobei vorzugsweise der vom extrudierten Mantel umfasste Kunststoff einen zumindest teil- oder abschnittsweise transparenten, transluzenten, opaken und/oder eingefärbten Kunststoff umfasst.

12. Lichtleiter (30) nach einem der Ansprüche 1 bis 11, aufweisend wenigstens eines der folgenden Merkmale:
- der Lichtleiter besteht aus flexiblen oder semi-flexiblen Faserbündeln (32) und der Mantel (31) ist zumindest teil- oder abschnittsweise als starre Hülle ausgeführt
- der Lichtleiter (30) besteht aus gezogenen Faserstäben und/oder gepressten Faserstäben und bildet einen starren Lichtleiter aus
- ein zuvor extrudierter Mantel (31) in bestimmten Abständen oder ein entsprechender Faserbündelabschnitt, welcher mit einem als Schlauch oder Schrumpfschlauch ausgebildetem Mantel (31) umgeben ist, ist entsprechend der finalen Bauteillänge geteilt und der Mantel (31) oder der Schlauch oder Schrumpfschlauch ist gegenüber dem Faserbündel (32) gelängt und die dabei entstehende Kavität mit optisch transparenten Kunststoff aufgefüllt oder ein vorgefertigtes klar transparentes Kunststoffteil oder ein Lichtleitstab oder Faserstab aus Glas oder Kunststoff ist in die Kavität eingesetzt und fixiert, wobei vorzugsweise der Mantelabschnitt, Schlauch- oder Schrumpfschlauchabschnitt, der die Kavität ausbildet, verformt ist und eine bestimmte Lichteintritts- oder Lichtaustrittskontur nach Aushärten des Kunststoffes oder nach Einsetzen des Kunststoffteils oder des Lichtleitstabs ausbildet.

13. Lichtleiter (30) nach einem der Ansprüche 1 bis 12,
wobei die proximalen und/ oder distalen Endflächen des Lichtleiters (30.1, 30.2) weitere aktive elektronische Elemente in Form von LEDs, Laser-Dioden, Sensoren oder Kamera-Chips aufweisen, welche an die terminierte Endfläche (30.1, 30.2) verbindbar und/oder mittels einer Rastverbindung ansteckbar sind.

14. Lichtleiter (30) nach einem der Ansprüche 1 bis 13,
wobei die proximalen und/ oder distalen Endflächen (30.1, 30.2) des Lichtleiters (30) als optisches Element zur Erzielung einer bestimmten Strahlformung ausgeführt sind, und dabei eine plane, konvexe, konkave Fläche oder eine hinsichtlich ihrer Topographie beliebig gestaltete Freiformfläche aufweisen.

15. Lichtleiter (30) nach einem der Ansprüche 1 bis 14,
mit einem Flächenverhältnis der Querschnittsfläche des Lichtleiters (30) zur Querschnittsfläche des Faserbündels (32) von mindestens 1,025 und höchstens 2,64.

16. Diagnose-, Operations- und/oder Therapiegerät (1), umfassend zumindest einen Lichtleiter (30) nach einem der Ansprüche 1 bis 15.

17. Verwendung des Lichtleiters (30) nach mindestens einem der vorstehenden Ansprüche 1 bis 15 in flexiblen oder starren Einweg-Endoskopen und/oder für In-Vitro-Diagnostik-Geräte.

18. Verwendung eines Lichtleiters (30) nach einem der Ansprüche 1 bis 15 in industriellen und/oder Konsumer-Produkten, beispielsweise als Beleuchtungslichtleiter bzw. Lichtleiter in Hausgeräten (Herde, Spülmaschinen, Kühl-/Gefrier-Schränke, Backöfen etc.) beziehungsweise Küchen-Klein-Geräte (Mixer, Toaster, Auftisch-Kochgeräten, Kaffee-Automaten etc.) zum Beispiel zur Kenntlichmachung von Betriebszuständen und/ oder zur Beleuchtung von Garräumen oder Innenräumen, insbesondere dann wenn diese mit Lebensmitteln in Kontakt kommen; Home-Ambiente-Beleuchtung; Automotive Exterieur-/Interieur-Beleuchtung; Maschinenbeleuchtung.
